# EUROPEAN PATENT SPECIFICATION

(11) **EP 2 220 506 B1**
(45) Date of publication and mention of the grant of the patent: **02.10.2013**
(21) Application number: 08843957.5
(22) Date of filing: 29.10.2008
(51) Int. Cl.: G01N 33/50, G01N 33/68, C12Q 1/34

(54) **BIOMARKERS FOR THE DETECTION OF EARLY STAGE OVARIAN CANCER**
BIOMARKER ZUM NACHWEIS VON OVARIALKARZINOM IM FRÜHSTADIUM
BIOMARQUEURS PERMETTANT LA DÉTECTION DU CANCER DES OVAIRES À UN STADE PRÉCOCE

(30) Priority: 29.10.2007 US 983378 P
(43) Date of publication of application: 25.08.2010
(73) Proprietor: Vermillion, Inc., Austin, TX 78731 (US); The Board of Regents,The University of Texas System, Austin, TX 78701 (US)
(72) Inventor: FUNG, Eric, Thomas, Los Altos, CA 94022 (US); CLARKE, Charlotte, Seabrook, TX 77586 (US); BAST, Robert, Houston, TX 77030 (US); COOMBES, Kevin, Houston, TX 77030 (US)
(74) Representative: Hiebl, Inge Elisabeth
(86) International application number: PCT/US2008/012323
(87) International publication number: WO 2009/058331

(56) References cited:
- WO-A1-2006/019906
- WO-A2-2005/098446
- WO-A2-2006/099126
- WO-A2-2007/002264
- WO-A2-2007/002535
- WO-A2-2008/048508
- US-A1- 2005 059 013
- US-A1- 2007 054 329
- FENG SU ET AL: "Validation of Candidate Serum Ovarian Cancer Biomarkers for Early Detection", BIOMARKER INSIGHTS, LIBERTAS ACADEMICA LTD, NEW ZEALAND, vol. 2, 1 January 2007 (2007-01-01), pages 369-375, XP007916996, ISSN: 1177-2719
- ZHANG Z ET AL: "THREE BIOMARKERS IDENTIFIED FROM SERUM PROTEOMIC ANALYSIS FOR THE DETECTION OF EARLY STAGE OVARIAN CANCER", CANCER RESEARCH, AMERICAN ASSOCIATION FOR CANCER REREARCH, US, vol. 64, no. 16, 15 August 2004 (2004-08-15), pages 5882-5890, XP001199859, ISSN: 0008-5472, DOI: DOI:10.1158/0008-5472.CAN-04-0746
- ROBERT E. GERSZTEN ET AL: 'The search for new cardiovascular biomarkers' NATURE vol. 451, no. 7181, 21 February 2008, pages 949 - 952, XP055033236 DOI: 10.1038/nature06802 ISSN: 0028-0836
- GREG P BERTENSHAW ET AL: "Multianalyte profiling of serum antigens and autoimmune and infectious disease molecules to identify biomarkers dysregulated in epithelial ovarian cancer", CANCER EPIDEMIOLOGY, BIOMARKERS AND PREVENTION, PHILADELPHIA, PA, US, vol. 17, no. 10, 1 October 2008 (2008-10-01), pages 2872-2881, XP002624682, ISSN: 1055-9965, DOI: 10.1158/1055-9965.EPI-08-0464

### SEQUENCE LISTING

<110> vermillion, Inc., et al.
<120> BIOMARKERS FOR THE DETECTION OF EARLY STAGE OVARIAN CANCER
<130> 21375EP
<140> EP 08 843 957.5
   <141> 2008-10-29
<150> US 2007/0983378
   <151> 2007-10-29
<160> 3
<170> PatentIn version 3.5
<210> 1
   <211> 30
   <212> PRT
   <213> Homo sapiens
<400> 1
   Met Asn Phe Arg Pro Gly Val Leu Ser Ser Arg Gln Leu Gly Leu Pro
   1               5                   10                  15
   Gly Pro Pro Asp Val Pro Asp His Ala Ala Tyr His Pro Phe
               20                  25                  30
<210> 2
   <211> 26
   <212> PRT
   <213> Homo sapiens
<400> 2
   Pro Gly Val Leu Ser Ser Arg Gln Leu Gly Leu Pro Gly Pro Pro Asp
   1 5 10 15
   Val Pro Asp His Ala Ala Tyr His Pro Phe
   20 25
<210> 3
   <211> 25
   <212> PRT
   <213> Homo sapiens
<400> 3
   Gly Val Leu Ser Ser Arg Gln Leu Gly Leu Pro Gly Pro Pro Asp Val
   1 5 10 15
   Pro Asp His Ala Ala Tyr His Pro Phe
   20 25

## Description

### FIELD OF THE INVENTION

The present invention relates to a method of determining if a subject has early stage ovarian cancer comprising: (a) measuring biomarkers in a sample from the subject and (b) correlating the measurement with the presence of ovarian cancer.

### BACKGROUND OF THE INVENTION

Ovarian cancer is among the most lethal gynecologic malignancies in developed countries. Annually in the United States alone, approximately 23,000 women are diagnosed with the disease and almost 14,000 women die from it. (Jamal, A., et al., CA Cancer J. Clin, 2002; 52:23-47). Despite progress in cancer therapy, ovarian cancer mortality has remained virtually unchanged over the past two decades. (Id.) Given the steep survival gradient relative to the stage at which the disease is diagnosed, early detection remains the most important factor in improving long-term survival of ovarian cancer patients.

The poor prognosis of ovarian cancer diagnosed at late stages, the cost and risk associated with confirmatory diagnostic procedures, and its relatively low prevalence in the general population together pose extremely stringent requirements on the sensitivity and specificity of a test for it to be used for screening for ovarian cancer in the general population.

The identification of tumor markers suitable for the early detection and diagnosis of cancer holds great promise to improve the clinical outcome of patients. It is especially important for patients presenting with vague or no symptoms or with tumors that are relatively inaccessible to physical examination. Despite considerable effort directed at early detection, no cost effective screening tests have been developed (Paley PJ., Curr Opin Oncol, 2001;13(5):399-402) and women generally present with disseminated disease at diagnosis. (Ozols RF, et al., Epithelial ovarian cancer. In: Hoskins WJ, Perez CA, Young RC, editors. Principles and Practice of Gynecologic Oncology. 3rd ed. Philadelphia: Lippincott, Williams and Wilkins; 2000. p. 981-1057).

The best-characterized tumor marker, CA125, is negative in approximately 30-40% of stage I ovarian carcinomas and its levels are elevated in a variety of benign diseases. (Meyer T, et al., Br J Cancer, 2000;82(9):1535-8; Buamah P., J Surg Oncol, 2000;75(4):264-5; Tuxen MK, et al., Cancer Treat Rev, 1995;21(3):215-45). Its use as a population-based screening tool for early detection and diagnosis of ovarian cancer is hindered by its low sensitivity and specificity. (MacDonald ND, et al., Eur J Obstet Gynecol Reprod Biol, 1999;82(2):155-7; Jacobs I, et al., Hum Reprod, 1989;4(1):1-12; Shih I-M, et al., Tumor markers in ovarian cancer. In: Diamandis EP, Fritsche, H., Lilja, H., Chan, D.W., and Schwartz, M., editor. Tumor markers physiology, pathobiology, technology and clinical applications. Philadelphia: AACC Press; in press). Although pelvic and more recently vaginal sonography has been used to screen high-risk patients, neither technique has the sufficient sensitivity and specificity to be applied to the general population. (MacDonald ND, et al., supra). Recent efforts in using CA125 in combination with additional tumor markers (Woolas RP XF, et al., J Natl Cancer Inst, 1993;85(21):1748-51; Woolas RP, et al., Gynecol Oncol, 1995;59(1):111-6; Zhang Z, et al., Gynecol Oncol, 1999;73(1):56-61; Zhang Z, et al., Use of Multiple Markers to Detect Stage I Epithelial Ovarian Cancers: Neural Network Analysis Improves Performance. American Society of Clinical Oncology 2001; Annual Meeting, Abstract) in a longitudinal risk of cancer model (Skates SJ, et al., Cancer, 1995;76(10 Suppl):2004-10), and in tandem with ultrasound as a second line test (Jacobs I DA, et al., Br Med J, 1993;306(6884):1030-34; Menon U TA, et al., British Journal of Obstetrics and Gynecology, 2000; 107(2): 165-69) have shown promising results in improving overall test specificity, which is critical for a disease such as ovarian cancer that has a relatively low prevalence.

Due to the dismal prognosis of late stage ovarian cancer, it is the general consensus that a physician will accept a test with a minimal positive predictive value of 10%. (Bast, R.C., et al., Cancer Treatment and Research, 2002; 107:61-97). Extending this to the general population, a general screening test would require a sensitivity greater than 70% and a specificity of 99.6%. Currently, none of the existing serologic markers, such as CA125, CA72-4, or M-CSF, individually delivers such a performance. (Bast, R.C., et al., Int J Biol Markers, 1998; 13:179-87).

FENG SU ET AL ("Validation of Candidate Serum Ovarian Cancer Biomarkers for Early Detection", BIOMARKER INSIGHTS, LIBERTAS ACADEMICA LTD, NEW ZEALAND, vol. 2, 1 January 2007 (2007-01-01), pages 369-375) discloses the measurement of the levels of CA125, ApoA1, TTR and TF in relation to their utility in early detection of ovarian cancer. It was found that the biomarker panel consisting of ApoA1, TTR and TF may significantly improve early detection of ovarian cancer.

ZHANG Z ET AL ("THREE BIOMARKERS IDENTIFIED FROM SERUM PROTEOMIC ANALYSIS FOR THE DETECTION OF EARLY STAGE OVARIAN CANCER", CANCER RESEARCH, AMERICAN ASSOCIATION FOR CANCER REREARCH, US, vol. 64, no. 16, 15 August 2004 (2004-08-15), pages 5882-5890) discloses the use of apolipoprotein A1, a truncated form of transthyretin and a cleavage fragment of inter-α-trypsin inhibitor heavy chain H4 for the detection of early stage invasive epithelial ovarian cancer.

Thus, there is a critical need to identify one or more panels of biomarkers that deliver the required sensitivity and specificity for early detection of ovarian cancer. Without an acceptable screening test, early detection remains the most critical factor in improving long-term survival of patients with ovarian cancer.

Thus, it is desirable to have a reliable and accurate method of determining the ovarian cancer status in patients, the results of which can then be used to manage subject treatment.

### SUMMARY

The present invention provides sensitive and quick methods that are useful for determining the early stage ovarian cancer status of patients or subjects by measuring and identifying particular biomarkers. The detection and measurement of these biomarkers in patient samples provides information that diagnosticians can correlate with the presence or absence of early stage ovarian cancer. The markers are characterized by mass/charge ratio, molecular weight and/or by their known protein identities. The markers can be resolved from other proteins in a sample by using a variety of fractionation techniques, *e.g.,* chromatographic separation coupled with mass spectrometry, protein capture using immobilized antibodies, bead-protein complexes or by traditional immunoassays. In preferred embodiments, the method of resolution involves Surface-Enhanced Laser Desorption/Ionization ("SELDI") mass spectrometry, in which the surface of the mass spectrometry probe comprises adsorbents that bind the markers.

More specifically, two panels of markers were identified herein. The first panel comprises (i) apolipoprotein A (ApoA1), (ii) transthyretin (TTR), (iii) inter-alpha (globulin) inhibitor H4 (plasma Kallikrein-sensitive glycoprotein) (ITIH4), (iv) transferrin (TFR) and (v) CA125. The second panel comprises (i) apolipoprotein A1 (ApoA1), (ii) transthyretin (TTR), (iii) CTAPIII, and (iv) CA125. These panels or markers are detecting early stage ovarian cancer, e.g., Stage 1 or Stage II.

The present invention provides an *in vitro* method of determining if an ovarian cancer patient has an early stage of ovarian cancer comprising:
(a) determining the concentration or expression levels or peak intensity values of a combination of biomarkers in a blood, serum, or plasma sample from the subject, wherein the biomarkers consist of: (i) apolipoprotein A1 (ApoA1), (ii) transthyretin (TTR), (iii) CTAPIII, and (iv) CA125; and
(b) correlating the measurement of the combination of biomarkers of a with ovarian cancer status, wherein the early stage ovarian cancer status is stage I or II ovarian cancer.

In certain embodiments, the methods further comprise managing subject treatment based on the status determined by the method. For example, if the result of the methods of the present invention is inconclusive or there is reason that confirmation of status is necessary, the physician may order more tests. Alternatively, if the status indicates that surgery is appropriate, the physician may schedule the patient for surgery. Furthermore, if the results show that treatment has been successful, no further management may be necessary.

The invention also provides for such methods where the panel of biomarkers is measured again after subject management. In these instances, the step of managing subject treatment is then repeated and/or altered depending on the result obtained.

For the mass values of the markers disclosed herein, the mass accuracy of the spectral instrument is considered to be about within +/- 0.15 percent of the disclosed molecular weight value. Additionally, to such recognized accuracy variations of the instrument, the spectral mass determination can vary within resolution limits of from about 400 to 1000 m/dm, where m is mass and dm is the mass spectral peak width at 0.5 peak height. Those mass accuracy and resolution variances associated with the mass spectral instrument and operation thereof are reflected in the use of the term "about" in the disclosure of the mass of each of four biomarkers. It is also intended that such mass accuracy and resolution variances and thus meaning of the term "about" with respect to the mass of each of the markers disclosed herein is inclusive of variants of the markers as may exist due to sex, genotype and/or ethnicity of the subject and the particular cancer or origin or stage thereof.

The accuracy of a diagnostic test is characterized by a Receiver Operating Characteristic curve ("ROC curve"). An ROC is a plot of the true positive rate against the false positive rate for the different possible cutpoints of a diagnostic test. An ROC curve shows the relationship between sensitivity and specificity. That is, an increase in sensitivity will be accompanied by a decrease in specificity. The closer the curve follows the left axis and then the top edge of the ROC space, the more accurate the test. Conversely, the closer the curve comes to the 45-degree diagonal of the ROC graph, the less accurate the test. The area under the ROC is a measure of test accuracy. The accuracy of the test depends on how well the test separates the group being tested into those with and without the disease in question. An area under the curve (referred to as "AUC") of 1 represents a perfect test, while an area of 0.5 represents a test of no use. Thus, preferred biomarkers and diagnostic methods of the present invention have an AUC greater than 0.50, more preferred tests have an AUC greater than 0.60, more preferred tests have an AUC greater than 0.70.

Preferred methods of measuring the biomarkers include use of a biochip array. Biochip arrays useful in the invention include protein and nucleic acid arrays. One or more markers are captured on the biochip array and subjected to laser ionization to detect the molecular weight of the markers. Analysis of the markers is, for example, by molecular weight of the one or more markers against a threshold intensity that is normalized against total ion current. Preferably, logarithmic transformation is used for reducing peak intensity ranges to limit the number of markers detected.

Another preferred method of measuring the biomarkers includes the use of a combinatorial ligand library synthesized on beads.

In preferred methods of the present invention, the step of correlating the measurement of the biomarkers with ovarian cancer status is performed by a software classification algorithm. Preferably, data is generated on immobilized subject samples on a biochip array, by subjecting said biochip array to laser ionization and detecting intensity of signal for mass/charge ratio; and, transforming the data into computer readable form; and executing an algorithm that classifies the data according to user input parameters, for detecting signals that represent markers present in ovarian cancer patients and are lacking in non-cancer subject controls.

Preferably the biochip surfaces are, for example, ionic, anionic, comprised of immobilized nickel ions, comprised of a mixture of positive and negative ions, comprised of one or more antibodies, single or double stranded nucleic acids, proteins, peptides or fragments thereof, amino acid probes, or phage display libraries.

In other preferred methods one or more of the markers are measured using laser desorption/ionization mass spectrometry, comprising providing a probe adapted for use with a mass spectrometer comprising an adsorbent attached thereto, and contacting the subject sample with the adsorbent, and; desorbing and ionizing the marker or markers from the probe and detecting the deionized/ionized markers with the mass spectrometer.

Preferably, the laser desorption/ionization mass spectrometry comprises: providing a substrate comprising an adsorbent attached thereto; contacting the subject sample with the adsorbent; placing the substrate on a probe adapted for use with a mass spectrometer comprising an adsorbent attached thereto; and, desorbing and ionizing the marker or markers from the probe and detecting the desorbed/ionized marker or markers with the mass spectrometer.

The adsorbent can for example be hydrophobic, hydrophilic, ionic or metal chelate adsorbent, such as, nickel or an antibody, single- or double stranded oligonucleotide, amino acid, protein, peptide or fragments thereof.

The methods of the present invention can be performed on any type of patient sample that would be amenable to such methods, e.g., blood, serum and plasma.

Described are kits comprising (a) capture reagents that bind a biomarkers comprising (i) apolipoprotein A1 (ApoA1), (ii) transthyretin (TTR), (iii) CTAPIII, and (iv) CA125; and (b) a container comprising the panel of biomarkers. While the capture reagent can be any type of reagent, preferably the reagent is a SELDI probe.

In certain kits of the present invention, the capture reagent comprises an immobilized metal chelate ("IMAC").

Certain kits further comprise a wash solution that selectively allows retention of the bound biomarker to the capture reagent as compared with other biomarkers after washing.

Described are kits comprising (a) capture reagents that binds a biomarkers comprising (i) apolipoprotein A1 (Apo1), (ii) transthyretin (TTR), (iii) CTAPIII, and (iv) CA125; and (b) instructions for using the capture reagent to measure the biomarker. In certain of these kits, the capture reagent comprises an antibody. Furthermore, some kits further comprise an MS probe to which the capture reagent is attached or is attachable. In some kits, the capture reagent comprises an IMAC. The kits may also contain a wash solution that selectively allows retention of the bound biomarker to the capture reagent as compared with other biomarkers after washing. Preferably, the kit comprises written instructions for use of the kit for determining ovarian cancer status and the instructions provide for contacting a test sample with the capture reagents and measuring one or more biomarkers retained by the capture reagents.

The kit also provides for capture reagents, which are antibodies, single or double stranded oligonucleotide, amino acid, protein, peptide or fragments thereof.

Measurement of one or more protein biomarkers using the kit, is by mass spectrometry or immunoassays such as an ELISA.

Purified proteins for detection of early stage ovarian cancer and/or generation of antibodies for further diagnostic assays are also provided for. Purified proteins include purified peptides of apolipoprotein A1 (ApoA1), transthyretin (TTR), CA125, or CTAPIII. Also disclosed are these purified peptides further comprising a detectable label.

Also disclosed is an article manufacture comprising capture reagents bound to the panel of biomarkers.

Disclosed is also a system comprising a plurality of capture reagents each of which has bound to it a different biomarker comprising
(i) apolipoprotein A1 (ApoA1), (ii) transthyretin (TTR), (iii) CTAPIII, and (iv) CA125.

Other aspects of the invention are described *infra.*

### BRIEF DESCRIPTION OF THE FIGURES

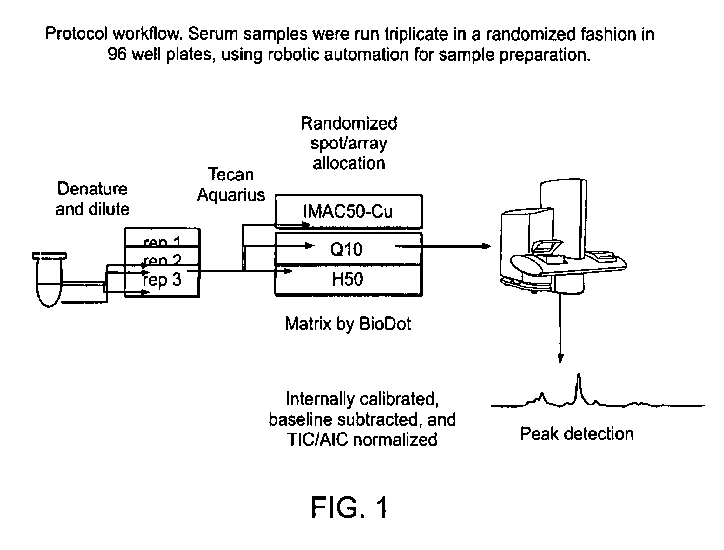

Figure 1 depicts the protocol workflow. Serum samples were run in triplicate in a randomized fashion in 96 well plates, using robotic automation for sample preparation.
Figure 2 depicts ROC curve plots for models trained on six subsets of data. The black dot represents CA125 performance alone. Performance of the models, however, was always assessed by comparing normal controls to early stage (I or II) ovarian cancer. Panel A depicts the proteomic markers without CA125, and Panel B depicts the proteomic markers with CA125.

### DEFINITIONS

Unless defined otherwise, all technical and scientific terms used herein have the meaning commonly understood by a person skilled in the art to which this invention belongs. The following references provide one of skill with a general definition of many of the terms used in this invention: Singleton et al., Dictionary of Microbiology and Molecular Biology (2nd ed. 1994); The Cambridge Dictionary of Science and Technology (Walker ed., 1988); The Glossary of Genetics, 5th Ed., R. Rieger et al. (eds.), Springer Verlag (1991); and Hale & Marham, The Harper Collins Dictionary of Biology (1991). As used herein, the following terms have the meanings ascribed to them unless specified otherwise.

"Gas phase ion spectrometer" refers to an apparatus that detects gas phase ions. Gas phase ion spectrometers include an ion source that supplies gas phase ions. Gas phase ion spectrometers include, for example, mass spectrometers, ion mobility spectrometers, and total ion current measuring devices. "Gas phase ion spectrometry" refers to the use of a gas phase ion spectrometer to detect gas phase ions.

"Mass spectrometer" refers to a gas phase ion spectrometer that measures a parameter that can be translated into mass-to-charge ratios of gas phase ions. Mass spectrometers generally include an ion source and a mass analyzer. Examples of mass spectrometers are time-of-flight, magnetic sector, quadrupole filter, ion trap, ion cyclotron resonance, electrostatic sector analyzer and hybrids of these. "Mass spectrometry" refers to the use of a mass spectrometer to detect gas phase ions.

"Laser desorption mass spectrometer" refers to a mass spectrometer that uses laser energy as a means to desorb, volatilize, and ionize an analyte.

"Tandem mass spectrometer" refers to any mass spectrometer that is capable of performing two successive stages of m/z-based discrimination or measurement of ions, including ions in an ion mixture. The phrase includes mass spectrometers having two mass analyzers that are capable of performing two successive stages of m/z-based discrimination or measurement of ions tandem-in-space. The phrase further includes mass spectrometers having a single mass analyzer that is capable of performing two successive stages of m/z-based discrimination or measurement of ions tandem-in-time. The phrase thus explicitly includes Qq-TOF mass spectrometers, ion trap mass spectrometers, ion trap-TOF mass spectrometers, TOF-TOF mass spectrometers, Fourier transform ion cyclotron resonance mass spectrometers, electrostatic sector - magnetic sector mass spectrometers, and combinations thereof.

"Mass analyzer" refers to a sub-assembly of a mass spectrometer that comprises means for measuring a parameter that can be translated into mass-to-charge ratios of gas phase ions. In a time-of-flight mass spectrometer the mass analyzer comprises an ion optic assembly, a flight tube and an ion detector.

"Ion source" refers to a sub-assembly of a gas phase ion spectrometer that provides gas phase ions. In one embodiment, the ion source provides ions through a desorption/ionization process. Such embodiments generally comprise a probe interface that positionally engages a probe in an interrogatable relationship to a source of ionizing energy (e.g., a laser desorption/ionization source) and in concurrent communication at atmospheric or subatmospheric pressure with a detector of a gas phase ion spectrometer.

Forms of ionizing energy for desorbing/ionizing an analyte from a solid phase include, for example: (1) laser energy; (2) fast atoms (used in fast atom bombardment); (3) high energy particles generated via beta decay of radionucleides (used in plasma desorption); and (4) primary ions generating secondary ions (used in secondary ion mass spectrometry). The preferred form of ionizing energy for solid phase analytes is a laser (used in laser desorption/ionization), in particular, nitrogen lasers, Nd-Yag lasers and other pulsed laser sources. "Fluence" refers to the energy delivered per unit area of interrogated image. A high fluence source, such as a laser, will deliver about 1 mJ / mm2 to 50 mJ / mm2. Typically, a sample is placed on the surface of a probe, the probe is engaged with the probe interface and the probe surface is struck with the ionizing energy. The energy desorbs analyte molecules from the surface into the gas phase and ionizes them.

Other forms of ionizing energy for analytes include, for example: (1) electrons that ionize gas phase neutrals; (2) strong electric field to induce ionization from gas phase, solid phase, or liquid phase neutrals; and (3) a source that applies a combination of ionization particles or electric fields with neutral chemicals to induce chemical ionization of solid phase, gas phase, and liquid phase neutrals.

"Solid support" refers to a solid material which can be derivatized with, or otherwise attached to, a capture reagent. Exemplary solid supports include probes, microtiter plates and chromatographic resins.

"Probe" in the context of this invention refers to a device adapted to engage a probe interface of a gas phase ion spectrometer (e.g., a mass spectrometer) and to present an analyte to ionizing energy for ionization and introduction into a gas phase ion spectrometer, such as a mass spectrometer. A "probe" will generally comprise a solid substrate (either flexible or rigid) comprising a sample presenting surface on which an analyte is presented to the source of ionizing energy.

"Biomarker panel" refers to one of the two biomarker panels set forth herein. A biomarker panel comprises (i) apolipoprotein A1 (ApoA1), (ii) transthyretin (TTR), (iii) inter-alpha (globulin) inhibitor H4 (plasma Kallikrein-sensitive glycoprotein) (ITIH4), (iv) transferrin (TFR) and (v) CA125. The biomarker panel according to the invention comprises (i) apolipoprotein A1 (ApoA1), (ii) transthyretin (TTR), (iii) CTAPIII, and (iv) CA125.

"Surface-enhanced laser desorption/ionization" or "SELDI" refers to a method of desorption/ionization gas phase ion spectrometry (e.g., mass spectrometry) in which the analyte is captured on the surface of a SELDI probe that engages the probe interface of the gas phase ion spectrometer. In "SELDI MS," the gas phase ion spectrometer is a mass spectrometer. SELDI technology is described in, e.g., U.S. patent 5,719,060 (Hutchens and Yip) and U.S. patent 6,225,047 (Hutchens and Yip).

"Surface-Enhanced Affinity Capture" or "SEAC" is a version of SELDI that involves the use of probes comprising an absorbent surface (a "SEAC probe"). "Adsorbent surface" refers to a surface to which is bound an adsorbent (also called a "capture reagent" or an "affinity reagent"). An adsorbent is any material capable of binding an analyte (e.g., a target polypeptide or nucleic acid). "Chromatographic adsorbent" refers to a material typically used in chromatography. Chromatographic adsorbents include, for example, ion exchange materials, metal chelators (e.g., nitriloacetic acid or iminodiacetic acid), immobilized metal chelates, hydrophobic interaction adsorbents, hydrophilic interaction adsorbents, dyes, simple biomolecules (e.g., nucleotides, amino acids, simple sugars and fatty acids) and mixed mode adsorbents (e.g., hydrophobic attraction/electrostatic repulsion adsorbents). "Biospecific adsorbent" refers an adsorbent comprising a biomolecule, e.g., a nucleic acid molecule (e.g., an aptamer), a polypeptide, a polysaccharide, a lipid, a steroid or a conjugate of these (e.g., a glycoprotein, a lipoprotein, a glycolipid, a nucleic acid (e.g., DNA)-protein conjugate). In certain instances the biospecific adsorbent can be a macromolecular structure such as a multiprotein complex, a biological membrane or a virus. Examples of biospecific adsorbents are antibodies, receptor proteins and nucleic acids. Biospecific adsorbents typically have higher specificity for a target analyte than chromatographic adsorbents. Further examples of adsorbents for use in SELDI can be found in U.S. Patent 6,225,047 (Hutchens and Yip, "Use of retentate chromatography to generate difference maps," May 1, 2001).

In some embodiments, a SEAC probe is provided as a pre-activated surface which can be modified to provide an adsorbent of choice. For example, certain probes are provided with a reactive moiety that is capable of binding a biological molecule through a covalent bond. Epoxide and carbodiimidizole are useful reactive moieties to covalently bind biospecific adsorbents such as antibodies or cellular receptors.

"Adsorption" refers to detectable non-covalent binding of an analyte to an adsorbent or capture reagent.

"Surface-Enhanced Neat Desorption" or "SEND" is a version of SELDI that involves the use of probes comprising energy absorbing molecules chemically bound to the probe surface. ("SEND probe.") "Energy absorbing molecules" ("EAM") refer to molecules that are capable of absorbing energy from a laser desorption/ ionization source and thereafter contributing to desorption and ionization of analyte molecules in contact therewith. The phrase includes molecules used in MALDI , frequently referred to as "matrix", and explicitly includes cinnamic acid derivatives, sinapinic acid ("SPA"), cyano-hydroxy-cinnamic acid ("CHCA") and dihydroxybenzoic acid, ferulic acid, hydroxyacetophenone derivatives, as well as others. It also includes EAMs used in SELDI. SEND is further described in United States patent 5,719,060.

"Surface-Enhanced Photolabile Attachment and Release" or "SEPAR" is a version of SELDI that involves the use of probes having moieties attached to the surface that can covalently bind an analyte, and then release the analyte through breaking a photolabile bond in the moiety after exposure to light, e.g., laser light. SEPAR is further described in United States patent 5,719,060.

"Eluant" or "wash solution" refers to an agent, typically a solution, which is used to affect or modify adsorption of an analyte to an adsorbent surface and/or remove unbound materials from the surface. The elution characteristics of an eluant can depend, for example, on pH, ionic strength, hydrophobicity, degree of chaotropism, detergent strength and temperature.

"Analyte" refers to any component of a sample that is desired to be detected. The term can refer to a single component or a plurality of components in the sample.

The "complexity" of a sample adsorbed to an adsorption surface of an affinity capture probe means the number of different protein species that are adsorbed.

"Molecular binding partners" and "specific binding partners" refer to pairs of molecules, typically pairs of biomolecules that exhibit specific binding. Molecular binding partners include, without limitation, receptor and ligand, antibody and antigen, biotin and avidin, and biotin and streptavidin.

"Monitoring" refers to recording changes in a continuously varying parameter.

"Biochip" refers to a solid substrate having a generally planar surface to which an adsorbent is attached. Frequently, the surface of the biochip comprises a plurality of addressable locations, each of which location has the adsorbent bound there. Biochips can be adapted to engage a probe interface and, therefore, function as probes.

"Protein biochip" refers to a biochip adapted for the capture of polypeptides. Many protein biochips are described in the art. These include, for example, protein biochips produced by Ciphergen Biosystems (Fremont, CA), Packard BioScience Company (Meriden CT), Zyomyx (Hayward, CA) and Phylos (Lexington, MA). Examples of such protein biochips are described in the following patents or patent applications: U.S. patent 6,225,047 (Hutchens and Yip, "Use of retentate chromatography to generate difference maps," May 1, 2001); International publication WO 99/51773 (Kuimelis and Wagner, "Addressable protein arrays," October 14, 1999); U.S. patent 6,329,209 (Wagner et al., "Arrays of protein-capture agents and methods of use thereof," December 11, 2001) and International publication WO 00/56934 (Englert et al., "Continuous porous matrix arrays," September 28, 2000).

Protein biochips produced by Ciphergen Biosystems comprise surfaces having chromatographic or biospecific adsorbents attached thereto at addressable locations. Ciphergen ProteinChip® arrays include NP20, H4, H50, SAX-2, WCX-2, CM-10, IMAC-3, IMAC-30, LSAX-30, LWCX-30, IMAC-40, PS-10, PS-20 and PG-20. These protein biochips comprise an aluminum substrate in the form of a strip. The surface of the strip is coated with silicon dioxide.

In the case of the NP-20 biochip, silicon oxide functions as a hydrophilic adsorbent to capture hydrophilic proteins.

H4, H50, SAX-2, WCX-2, CM-10, IMAC-3, IMAC-30, PS-10 and PS-20 biochips further comprise a functionalized, cross-linked polymer in the form of a hydrogel physically attached to the surface of the biochip or covalently attached through a silane to the surface of the biochip. The H4 biochip has isopropyl functionalities for hydrophobic binding. The H50 biochip has nonylphenoxy-poly(ethylene glycol)methacrylate for hydrophobic binding. The SAX-2 biochip has quaternary ammonium functionalities for anion exchange. The WCX-2 and CM-10 biochips have carboxylate functionalities for cation exchange. The IMAC-3 and IMAC-30 biochips have nitriloacetic acid functionalities that adsorb transition metal ions, such as Cu++ and Ni++, by chelation. These immobilized metal ions allow adsorption of peptide and proteins by coordinate bonding. The PS-10 biochip has carboimidizole functional groups that can react with groups on proteins for covalent binding. The PS-20 biochip has epoxide functional groups for covalent binding with proteins. The PS-series biochips are useful for binding biospecific adsorbents, such as antibodies, receptors, lectins, heparin, Protein A, biotin/streptavidin and the like, to chip surfaces where they function to specifically capture analytes from a sample. The PG-20 biochip is a PS-20 chip to which Protein G is attached. The LSAX-30 (anion exchange), LWCX-30 (cation exchange) and IMAC-40 (metal chelate) biochips have functionalized latex beads on their surfaces. Such biochips are further described in: WO 00/66265 (Rich et al., "Probes for a Gas Phase Ion Spectrometer," November 9, 2000); WO 00/67293 (Beecher et al., "Sample Holder with Hydrophobic Coating for Gas Phase Mass Spectrometer," November 9, 2000); U.S. patent application US20030032043A1 (Pohl and Papanu, "Latex Based Adsorbent Chip," July 16, 2002).

Upon capture on a biochip, analytes can be detected by a variety of detection methods selected from, for example, a gas phase ion spectrometry method, an optical method, an electrochemical method, atomic force microscopy and a radio frequency method. Gas phase ion spectrometry methods are described herein. Of particular interest is the use of mass spectrometry and, in particular, SELDI. Optical methods include, for example, detection of fluorescence, luminescence, chemiluminescence, absorbance, reflectance, transmittance, birefringence or refractive index (e.g., surface plasmon resonance, ellipsometry, a resonant mirror method, a grating coupler waveguide method or interferometry). Optical methods include microscopy (both confocal and non-confocal), imaging methods and non-imaging methods. Immunoassays in various formats (e.g., ELISA) are popular methods for detection of analytes captured on a solid phase. Electrochemical methods include voltametry and amperometry methods. Radio frequency methods include multipolar resonance spectroscopy.

"Marker" in the context of the present invention refers to a polypeptide (of a particular apparent molecular weight), which is differentially present in a sample taken from patients having human cancer as compared to a comparable sample taken from control subjects (*e.g.,* a person with a negative diagnosis or undetectable cancer, normal or healthy subject). The term "biomarker" is used interchangeably with the term "marker."

The term "measuring" means methods which include detecting the presence or absence of marker(s) in the sample, quantifying the amount of marker(s) in the sample, and/or qualifying the type of biomarker. Measuring can be accomplished by methods known in the art and those further described herein, including but not limited to SELDI and immunoassay. Any suitable methods can be used to detect and measure one or more of the markers described herein. These methods include, without limitation, mass spectrometry (*e.g.,* laser desorption/ionization mass spectrometry), fluorescence (*e.g.* sandwich immunoassay), surface plasmon resonance, ellipsometry and atomic force microscopy.

The phrase "differentially present" refers to differences in the quantity and/or the frequency of a marker present in a sample taken from patients having human cancer as compared to a control subject. For example, the IAIH4 fragment is present at an elevated level in samples of ovarian cancer patients compared to samples from control subjects. In contrast, Apo A1 and transthyretin described herein are present at a decreased level in samples of ovarian cancer patients compared to samples from control subjects. Furthermore, a marker can be a polypeptide, which is detected at a higher frequency or at a lower frequency in samples of human cancer patients compared to samples of control subjects. A marker can be differentially present in terms of quantity, frequency or both.

A polypeptide is differentially present between two samples if the amount of the polypeptide in one sample is statistically significantly different from the amount of the polypeptide in the other sample. For example, a polypeptide is differentially present between the two samples if it is present at least about 120%, at least about 130%, at least about 150%, at least about 180%, at least about 200%, at least about 300%, at least about 500%, at least about 700%, at least about 900%, or at least about 1000% greater than it is present in the other sample, or if it is detectable in one sample and not detectable in the other.

Alternatively or additionally, a polypeptide is differentially present between two sets of samples if the frequency of detecting the polypeptide in the ovarian cancer patients' samples is statistically significantly higher or lower than in the control samples. For example, a polypeptide is differentially present between the two sets of samples if it is detected at least about 120%, at least about 130%, at least about 150%, at least about 180%, at least about 200%, at least about 300%, at least about 500%, at least about 700%, at least about 900%, or at least about 1000% more frequently or less frequently observed in one set of samples than the other set of samples.

"Diagnostic" means identifying the presence or nature of a pathologic condition, i.e., ovarian cancer. Diagnostic methods differ in their sensitivity and specificity. The "sensitivity" of a diagnostic assay is the percentage of diseased individuals who test positive (percent of "true positives"). Diseased individuals not detected by the assay are "false negatives." Subjects who are not diseased and who test negative in the assay, are termed "true negatives." The "specificity" of a diagnostic assay is 1 minus the false positive rate, where the "false positive" rate is defined as the proportion of those without the disease who test positive. While a particular diagnostic method may not provide a definitive diagnosis of a condition, it suffices if the method provides a positive indication that aids in diagnosis.

A "test amount" of a marker refers to an amount of a marker present in a sample being tested. A test amount can be either in absolute amount (*e.g.,* µg/ml) or a relative amount (*e.g.,* relative intensity of signals).

A "diagnostic amount" of a marker refers to an amount of a marker in a subject's sample that is consistent with a diagnosis of ovarian cancer. A diagnostic amount can be either in absolute amount (*e.g.,* µg/ml) or a relative amount (*e.g.,* relative intensity of signals).

A "control amount" of a marker can be any amount or a range of amount, which is to be compared against a test amount of a marker. For example, a control amount of a marker can be the amount of a marker in a person without ovarian cancer. A control amount can be either in absolute amount (*e.g.,* µg/ml) or a relative amount (*e.g.,* relative intensity of signals).

"Antibody" refers to a polypeptide ligand substantially encoded by an immunoglobulin gene or immunoglobulin genes, or fragments thereof, which specifically binds and recognizes an epitope (*e.g.,* an antigen). The recognized immunoglobulin genes include the kappa and lambda light chain constant region genes, the alpha, gamma, delta, epsilon and mu heavy chain constant region genes, and the myriad immunoglobulin variable region genes. Antibodies exist, *e.g.,* as intact immunoglobulins or as a number of well-characterized fragments produced by digestion with various peptidases. This includes, *e.g.*, Fab' and F(ab)'₂ fragments. The term "antibody," as used herein, also includes antibody fragments either produced by the modification of whole antibodies or those synthesized de novo using recombinant DNA methodologies. It also includes polyclonal antibodies, monoclonal antibodies, chimeric antibodies, humanized antibodies, or single chain antibodies. "Fc" portion of an antibody refers to that portion of an immunoglobulin heavy chain that comprises one or more heavy chain constant region domains, CH₁, CH₂ and CH₃, but does not include the heavy chain variable region.

"Managing subject treatment" refers to the behavior of the clinician or physician subsequent to the determination of ovarian cancer status. For example, if the result of the methods of the present invention is inconclusive or there is reason that confirmation of status is necessary, the physician may order more tests. Alternatively, if the status indicates that surgery is appropriate, the physician may schedule the patient for surgery. Likewise, if the status is negative, e.g., late stage ovarian cancer or if the status is acute, no further action may be warranted. Furthermore, if the results show that treatment has been successful, no further management may be necessary.

The term "stage" or "cancer stage" is intended to mean a classification of ovarian cancer that is based on the size, invasiveness, progression, migration, etc. of cancer in a subject. The stages of ovarian cancer are well defined. Stage I refers to ovarian cancer wherein the cancer is still contained within the ovary (or ovaries). Specifically, stage IA cancer has developed in one ovary, and the tumor is confined to the inside of the ovary. There is no cancer on the outer surface of the ovary. Laboratory examination of washings from the abdomen and pelvis did not find any cancer cells. Stage IB cancer has developed within both ovaries without any tumor on their outer surfaces. Laboratory examination of washings from the abdomen and pelvis did not find any cancer cells. Stage IC cancer is present in one or both ovaries and 1 or more of the following are present: cancer on the outer surface of at least one of the ovaries; in the case of cystic tumors (fluid-filled tumors), the capsule (outer wall of the tumor) has ruptured (burst); or laboratory examination found cancer cells in fluid or washings from the abdomen.

Stage II cancer is in one or both ovaries and has involved other organs (such as the uterus, fallopian tubes, bladder, the sigmoid colon, or the rectum) within the pelvis. Specifically, stage IIA cancer has spread to or has actually invaded the uterus or the fallopian tubes, or both. Laboratory examination of washings from the abdomen did not find any cancer cells. Stage IIB cancer has spread to other nearby pelvic organs such as the bladder, the sigmoid colon, or the rectum. Laboratory examination of fluid from the abdomen did not find any cancer cells. Stage IIC cancer has spread to pelvic organs as in stages IIA or IIB and laboratory examination of the washings from the abdomen found evidence of cancer cells.

Stage III cancer involves 1 or both ovaries, and 1 or both of the following are present: (1) cancer has spread beyond the pelvis to the lining of the abdomen; (2) cancer has spread to lymph nodes. The cancer is Stage IIIA if, during the staging operation, the surgeon can see cancer involving the ovary or ovaries, but no cancer is grossly visible (can be seen without using a microscope) in the abdomen and the cancer has not spread to lymph nodes. However, when biopsies are checked under a microscope, tiny deposits of cancer are found in the lining of the upper abdomen. Stage IIIB cancer is in one or both ovaries, and deposits of cancer large enough for the surgeon to see, but smaller than 2 cm (about 3/4 inch) across, are present in the abdomen. Cancer has not spread to the lymph nodes. For a cancer to be stage IIIC the cancer is in one or both ovaries, and one or both of the following are present: cancer has spread to lymph nodes and/or deposits of cancer larger than 2 cm (about 3/4 inch) across are seen in the abdomen.

Stage IV cancer is the most advanced stage of ovarian cancer. The cancer is in one or both ovaries. Distant metastasis (spread of the cancer to the inside of the liver, the lungs, or other organs located outside of the peritoneal cavity) has occurred. Finding ovarian cancer cells in pleural fluid (from the cavity that surrounds the lungs) is also evidence of stage IV disease.

As used herein, the term "recurrent ovarian cancer" is intended to mean that the disease has come back (recurred) after completion of treatment.

### DETAILED DESCRIPTION OF THE INVENTION

The present invention provides sensitive and quick methods that are useful for determining if a patient has early stage ovarian cancer by measuring and identifying particular biomarkers. The panel of biomarkers identified herein is useful to see if a subject has early stage ovarian cancer. The detection and measurement of these biomarkers in patient samples provides information that diagnosticians can correlate to the cancer status of a patient.

More specifically, two panels of biomarkers were discovered and characterized, in accordance with the methods described herein as. The first panel (referential) comprises (i) apolipoprotein A1 (ApoA1), (ii) transthyretin (TTR), (iii) inter-alpha (globulin) inhibitor H4 (plasma Kallikrein-sensitive glycoprotein) (ITIH4), (iv) transferrin (TFR) and (v) CA125. The second biomarker panel (according to the invention) comprises (i) apolipoprotein A1 (ApoA1), (ii) transthyretin (TTR), (iii) CTAPIII, and (iv) CA125.

Some of these biomarkers have been disclosed in PCT/US2005/010783 (WO 2005/098447); US Patent Application Publication 2005/0059013; PCT/US03/00531 (WO03/057014); PCT/US2003/024636 (WO 2004/012588).

These biomarkers assess a patient's ovarian cancer status after having developed ovarian cancer and could potentially provide additional information to physicians for clinical decision-making. This is supported by Receiver-Operating Characteristic (ROC) curve analysis in an independent validation. For example, several large-scale studies have suggested that ovarian cancer patients with surgical procedures operated by gynecological oncologists tend to have a better long-term survival. However, other studies concluded that currently only about one third of ovarian cancer patients undergoing surgical procedures in the US are treated by gynecological oncologists. With the current total number of gynecological oncologists available, it is still not practical to have all patients undergoing surgery for suspected ovarian cancer be operated by gynecologic oncologists. The biomarkers have the potential to be used to identify patients with the lower probability of surviving ovarian cancer and recommend them for treatment by gynecologic oncologists.

High-throughput protein profiling combined with effective use of bioinformatics tools provides a useful approach to screening for cancer markers. Briefly, the system used in the present invention utilizes chromatographic ProteinChip^{®} Arrays to assay samples using SELDI (Surface Enhanced Laser Desorption/Ionization). Proteins bound to the arrays are read in a ProteinChip^{®} Reader, a time-of-flight mass spectrometer.

The present invention is based upon the discovery of protein markers that are differentially present in samples of ovarian cancer patients and control subjects, and the application of this discovery in methods and kits for determining ovarian cancer status. The panel of markers described herein is particularly useful in diagnosing early stage ovarian cancer. These protein markers are found in samples from ovarian cancer patients at levels that are different than the levels in samples from women in whom human cancer is undetectable. Accordingly, the amount of these biomarkers found in a test sample compared to a control, or the presence or absence of one or more markers in the test sample provides useful information regarding the ovarian cancer status of the patient.

### I. DESCRIPTION OF THE BIOMARKERS

### 1. IAIH4 FRAGMENTS (referential)

One or more of a closely related set of cleavage fragments of inter-α-trypsin inhibitor heavy chain H4 precursor, also referred to alternatively herein as "ITIH4 fragments." ITIH4 fragments are described as biomarkers for ovarian cancer in US patent publication 2005-0059013 A1, International Patent Publication WO 2005/098447 and Fung et al., Int. J. Cancer 115:783-789 (2005). ITIH4 fragments can be selected from the group consisting of ITIH4 fragment no. 1, ITIH4 fragment no. 2, and ITIH4 fragment no. 3.

The amino acid sequences of the ITIH4 fragments were determined to be: ITIH4 fragment 1 (SEQ ID NO: 5): MNFRPGVLSSRQLGLPGPPDVPDHAAYHPF ITIH4 fragment 2 (SEQ ID NO: 6): PGVLSSRQLGLPGPPDVPDHAAYHPF ITIH4 fragment 3 (SEQ ID NO: 7): GVLSSRQLGLPGPPDVPDHAAYHPF.

ITIH4 precursor is a 930 amino acid protein (SwissProt Q14624). ITIH4 fragment 1 spans amino acids 658-687 of human ITIH4 precursor. ITIH4 fragment 2 spans amino acids 662-687 of ITIH4 precursor. ITIH4 fragment 3 spans amino acids 663-687 of ITIH4 precursor.

Additionally, disclosed are modified forms of ITIH4 fragment. Modification of ITIH4 fragment may include the post-translational addition of various chemical groups, for example, glycosylation, lipidation, cysteinylation, and glutathionylation.

### 2. CTAPIII

A biomarker that is useful in the methods of the present invention is CTAP-III (connective tissue activating peptide III), derived from platelet basic protein. CTAPIII is described as a biomarker for ovarian cancer in US provisional patent application 60/693,324, filed June 22, 2005 (Zhang et al.). CTAP-III is an 85 amino acid protein (SwissProt P02775)(SEQ ID NO: 8). CTAP-III is recognized by antibodies available from, e.g., Chemicon International (catalog 1484P) (www.chemicon.com, Temecula, CA.) CTAP-III is a fragment of platelet basic protein and includes amino acids 44-128 of platelet basic protein.

### 3. TRANSTHYRETIN

Transthyretin, also called "pre-albumin" is another biomarker that is useful in the methods of the present invention. Transthyretin and variants thereof are described as biomarkers for ovarian cancer in US patent publication 2005-0059013 A1 and International Patent Publication WO 2005/098447. Unmodified transthyretin is a 127 amino acid protein deriving from a 147 amino acid precursor (SwissProt Accession No. P02766) (SEQ ID NO: 9). The transthyretin biomarkers of the present invention include any or all of unmodified transthyretin and various modified forms. Transthyretin is recognized by antibodies available from, e.g., Dako (catalog A0002) (www.dako.com, Glostrup, Denmark).

In mass spectra of serum, transthyretin appears as a cluster of peaks around 13.9K Daltons. This cluster includes several forms of transthyretin including unmodified transthyretin, S-sulfonated thransthyretin, S-cysteinylated transthyretin, S-Gly-Cys transthyretin and S-glutathionylated transthryetin. Any and/or all of these is useful as a biomarker for ovarian cancer. However, the S-cysteinylated version represents the dominant form in the spectrum and is a preferred biomarker when using mass spectrometry. Another variant of transthyretin useful as a biomarker is transthyretin ΔN10.

### 4. TRANSFERRIN (referential)

Another biomarker is transferrrin. Transferrrin is described as a biomarker for ovarian cancer in US patent publication 2005-0214760 A1. Transferrrin is a 679 amino acid protein derived from a 698 amino acid precursor (GenBank Accession No. NP_001054 GI:4557871; SwissProt Accesion No. P02787) (SEQ ID NO: 10). Transferrrin is recognized by antibodies available from, e.g., Dako (catalog A006) (www.dako.com, Glostrup, Denmark). Transferrin is glycosylated. Therefore, the measured molecular weight is higher than the theoretical weight, which does not take glycosylation into account.

### 5. APOLIPOPROTEIN A1

Another biomarker that is useful in the methods of the present invention is apolipoprotein A1, also referred to as Apo A1. Apo A1 is described as a biomarker for ovarian cancer in US patent publication 2005-0059013 A1 and International Patent Publication WO 2005/098447. Apo A1 is a 243 amino acid protein derived from a 267 amino acid precursor (SwissProt Accession No. P02647) SEQ ID NO: 12). Apo A1 is recognized by antibodies available from, e.g., EMD Biosciences, Inc. (catalog 178474) (www.emdbiosciences.com/home.asp, San Diego, CA). ApoA1 can be visualized on H50 arrays or IMAC30 or IMAC50 arrays, but is preferentially visualized on H50 arrays.

Preferred methods of the present invention include the use of modified forms of Apo A1, such as C-terminal truncation of Apo AI (amino acids 1 thru 190-200). Modification of Apo A1 may include the post-translational addition of various chemical groups, for example, glycosylation and lipidation.

Because the biomarker of this invention is characterized by mass-to-charge ratio, binding properties and spectral shape, they can be detected by mass spectrometry without knowing their specific identity. However, if desired, biomarkers whose identity is not determined can be identified by, for example, determining the amino acid sequence of the polypeptides. For example, a biomarker can be peptide-mapped with a number of enzymes, such as trypsin or V8 protease, and the molecular weights of the digestion fragments can be used to search databases for sequences that match the molecular weights of the digestion fragments generated by the various enzymes. Alternatively, protein biomarkers can be sequenced using tandem MS technology. In this method, the protein is isolated by, for example, gel electrophoresis. A band containing the biomarker is cut out and the protein is subject to protease digestion. Individual protein fragments are separated by a first mass spectrometer. The fragment is then subjected to collision-induced cooling, which fragments the peptide and produces a polypeptide ladder. A polypeptide ladder is then analyzed by the second mass spectrometer of the tandem MS. The difference in masses of the members of the polypeptide ladder identifies the amino acids in the sequence. An entire protein can be sequenced this way, or a sequence fragment can be subjected to database mining to find identity candidates.

It has been found that proteins frequently exist in a sample in a plurality of different forms characterized by a detectably different mass. These forms can result from either, or both, of pre- and post-translational modification. Pre-translational modified forms include allelic variants, slice variants and RNA editing forms. Post-translationally modified forms include forms resulting from proteolytic cleavage (e.g., fragments of a parent protein), glycosylation, phosphorylation, lipidation, oxidation, methylation, cystinylation, sulphonation and acetylation. The collection of proteins including a specific protein and all modified forms of it is referred to herein as a "protein cluster." The collection of all modified forms of a specific protein, excluding the specific protein, itself, is referred to herein as a "modified protein cluster." Modified forms of the biomarker of this invention also may be used, themselves, as biomarkers. In certain cases the modified forms may exhibit better discriminatory power in diagnosis than the specific forms set forth herein.

Modified forms of a biomarker can be initially detected by any methodology that can detect and distinguish the modified from the biomarker. A preferred method for initial detection involves first capturing the biomarker and modified forms of it, e.g., with biospecific capture reagents, and then detecting the captured proteins by mass spectrometry. More specifically, the proteins are captured using biospecific capture reagents, such as antibodies, aptamers or Affibodies that recognize the biomarker and modified forms of it. This method also will also result in the capture of protein interactors that are bound to the proteins or that are otherwise recognized by antibodies and that, themselves, can be biomarkers. In certain embodiments, the biospecific capture reagents are bound to a solid phase. Then, the captured proteins can be detected by SELDI mass spectrometry or by eluting the proteins from the capture reagent and detecting the eluted proteins by traditional MALDI or by SELDI. The use of mass spectrometry is especially attractive because it can distinguish and quantify modified forms of a protein based on mass and without the need for labeling.

Preferably, the biospecific capture reagent is bound to a solid phase, such as a bead, a plate, a membrane or a chip. Methods of coupling biomolecules, such as antibodies, to a solid phase are well known in the art. They can employ, for example, bifunctional linking agents, or the solid phase can be derivatized with a reactive group, such as an epoxide or an imidizole, that will bind the molecule on contact. Biospecific capture reagents against different target proteins can be mixed in the same place, or they can be attached to solid phases in different physical or addressable locations. For example, one can load multiple columns with derivatized beads, each column able to capture a single protein cluster. Alternatively, one can pack a single column with different beads derivatized with capture reagents against a variety of protein clusters, thereby capturing all the analytes in a single place. Accordingly, antibody-derivatized bead-based technologies, such as xMAP technology of Luminex (Austin, TX) can be used to detect the protein clusters. However, the biospecific capture reagents must be specifically directed toward the members of a cluster in order to differentiate them.

In yet another embodiment, the surfaces of biochips can be derivatized with the capture reagents directed against protein clusters either in the same location or in physically different addressable locations. One advantage of capturing different clusters in different addressable locations is that the analysis becomes simpler.

After identification of modified forms of a protein and correlation with the clinical parameter of interest, the modified form can be used as a biomarker in any of the methods of this invention. At this point, detection of the modified form can be accomplished by any specific detection methodology including affinity capture followed by mass spectrometry, or traditional immunoassay directed specifically the modified form. Immunoassay requires biospecific capture reagents, such as antibodies, to capture the analytes. Furthermore, if the assay must be designed to specifically distinguish protein and modified forms of protein. This can be done, for example, by employing a sandwich assay in which one antibody captures more than one form and second, distinctly labeled antibodies, specifically bind, and provide distinct detection of, the various forms. Antibodies can be produced by immunizing animals with the biomolecules. This invention contemplates traditional immunoassays including, for example, sandwich immunoassays including ELISA or fluorescence-based immunoassays, as well as other enzyme immunoassays.

### II. TEST SAMPLES

### A) SUBJECT TYPES

Samples are collected from subjects, e.g., women, who want to establish ovarian cancer status. The subjects may be women showing no health issues or predisposition to cancer and are being tested as part of a normal examination. The subjects may be women who have been determined to have a high risk of ovarian cancer based on their family history. Other patients include women who have ovarian cancer or women diagnosed with a pelvic mass and the test is being used to determine the effectiveness of therapy or treatment they are receiving. Also, patients could include healthy women who are having a test as part of a routine examination, or to establish baseline levels of the biomarkers. Samples may be collected from women who had been diagnosed with ovarian cancer and received treatment to eliminate the cancer, or perhaps are in remission.

### B) TYPES OF SAMPLE AND PREPARATION OF THE SAMPLE

The markers can be measured in different types of biological samples. The sample is preferably a biological fluid sample. Examples of a biological fluid sample useful in this invention include blood, blood serum, plasma, vaginal secretions, urine, ovarian cyst fluid, tears, saliva, *etc.* Because all of the markers are found in blood serum, blood serum is a preferred sample source for embodiments of the invention.

If desired, the sample can be prepared to enhance detectability of the markers. For example, to increase the detectability of markers, a blood serum sample from the subject can be preferably fractionated by, *e.g.,* Cibacron blue agarose chromatography and single stranded DNA affinity chromatography, anion exchange chromatography, affinity chromatography (e.g., with antibodies) and the like. The method of fractionation depends on the type of detection method used. Any method that enriches for the protein of interest can be used. Sample preparations, such as pre-fractionation protocols, are optional and may not be necessary to enhance detectability of markers depending on the methods of detection used. For example, sample preparation may be unnecessary if antibodies that specifically bind markers are used to detect the presence of markers in a sample.

Typically, sample preparation involves fractionation of the sample and collection of fractions determined to contain the biomarkers. Methods of pre-fractionation include, for example, size exclusion chromatography, ion exchange chromatography, heparin chromatography, affinity chromatography, sequential extraction, gel electrophoresis and liquid chromatography. The analytes also may be modified prior to detection. These methods are useful to simplify the sample for further analysis. For example, it can be useful to remove high abundance proteins, such as albumin, from blood before analysis. Examples of methods of fractionation are described disclosure.

Preferably, the sample is pre-fractionated by anion exchange chromatography. Anion exchange chromatography allows pre-fractionation of the proteins in a sample roughly according to their charge characteristics. For example, a Q anion-exchange resin can be used (*e.g.,* Q HyperD F, Biosepra), and a sample can be sequentially eluted with eluants having different pH's. Anion exchange chromatography allows separation of biomolecules in a sample that are more negatively charged from other types of biomolecules. Proteins that are eluted with an eluant having a high pH is likely to be weakly negatively charged, and a fraction that is eluted with an eluant having a low pH is likely to be strongly negatively charged. Thus, in addition to reducing complexity of a sample, anion exchange chromatography separates proteins according to their binding characteristics.

In preferred embodiments, the serum samples are fractionated via anion exchange chromatography. Signal suppression of lower abundance proteins by high abundance proteins presents a significant challenge to SELDI mass spectrometry. Fractionation of a sample reduces the complexity of the constituents of each fraction. This method can also be used to attempt to isolate high abundance proteins into a fraction, and thereby reduce its signal suppression effect on lower abundance proteins. Anion exchange fractionation separates proteins by their isoelectric point (pI). Proteins are comprised of amino acids, which are ambivalent-their charge changes based on the pH of the environment to which they are exposed. A protein's pI is the pH at which the protein has no net charge. A protein assumes a neutral charge when the pH of the environment is equivalent to pI of the protein. When the pH rises above the pI of the protein, the protein assumes a net negative charge. Similarly, when the pH of the environment falls below the pI of the protein, the protein has a net positive charge. The serum samples were fractionated according to the protocol set forth in the Examples below to obtain the markers described herein.

After capture on anion exchange, proteins were eluted in a series of step washes at pH 9, pH 7, pH 5, pH 4 and pH 3. A panel of three potential biomarkers was discovered by UMSA analysis of profiling data of three fractions (pH 9/flow through, pH 4, and organic solvent). Two of the peaks were from fraction pH 4 at m/z of 12828 and 28043, both down-regulated in the cancer group, and the third was from fraction pH 9/flow through at m/z of 3272, up-regulated in the cancer group. All bound to the immobilized metal affinity chromatography array charged with copper ions (IMAC3-Cu).

Biomolecules in a sample can also be separated by high-resolution electrophoresis, *e.g.,* one or two-dimensional gel electrophoresis. A fraction containing a marker can be isolated and further analyzed by gas phase ion spectrometry. Preferably, two-dimensional gel electrophoresis is used to generate two-dimensional array of spots of biomolecules, including one or more markers. *See, e.g.,* Jungblut and Thiede, Mass Spectr. Rev. 16:145-162 (1997).

The two-dimensional gel electrophoresis can be performed using methods known in the art. *See, e.g.,* Deutscher ed., Methods In Enzymology vol. 182. Typically, biomolecules in a sample are separated by, *e.g.,* isoelectric focusing, during which biomolecules in a sample are separated in a pH gradient until they reach a spot where their net charge is zero (*i.e.,* isoelectric point). This first separation step results in one-dimensional array of biomolecules. The biomolecules in one-dimensional array is further separated using a technique generally distinct from that used in the first separation step. For example, in the second dimension, biomolecules separated by isoelectric focusing are further separated using a polyacrylamide gel, such as polyacrylamide gel electrophoresis in the presence of sodium dodecyl sulfate (SDS-PAGE). SDS-PAGE gel allows further separation based on molecular mass of biomolecules. Typically, two-dimensional gel electrophoresis can separate chemically different biomolecules in the molecular mass range from 1000-200,000 Da within complex mixtures. The pI range of these gels is about 3-10 (wide range gels).

Biomolecules in the two-dimensional array can be detected using any suitable methods known in the art. For example, biomolecules in a gel can be labeled or stained (*e.g.,* Coomassie Blue or silver staining). If gel electrophoresis generates spots that correspond to the molecular weight of one or more markers of the invention, the spot can be further analyzed by gas phase ion spectrometry. For example, spots can be excised from the gel and analyzed by gas phase ion spectrometry. Alternatively, the gel containing biomolecules can be transferred to an inert membrane by applying an electric field. Then a spot on the membrane that approximately corresponds to the molecular weight of a marker can be analyzed by gas phase ion spectrometry. In gas phase ion spectrometry, the spots can be analyzed using any suitable techniques, such as MALDI or SELDI (*e.g.,* using ProteinChip^{®} array) as described herein.

Prior to gas phase ion spectrometry analysis, it may be desirable to cleave biomolecules in the spot into smaller fragments using cleaving reagents, such as proteases (*e.g.,* trypsin). The digestion of biomolecules into small fragments provides a mass fingerprint of the biomolecules in the spot, which can be used to determine the identity of markers if desired.

High performance liquid chromatography (HPLC) can also be used to separate a mixture of biomolecules in a sample based on their different physical properties, such as polarity, charge and size. HPLC instruments typically consist of a reservoir of mobile phase, a pump, an injector, a separation column, and a detector. Biomolecules in a sample are separated by injecting an aliquot of the sample onto the column. Different biomolecules in the mixture pass through the column at different rates due to differences in their partitioning behavior between the mobile liquid phase and the stationary phase. A fraction that corresponds to the molecular weight and/or physical properties of one or more markers can be collected. The fraction can then be analyzed by gas phase ion spectrometry to detect markers. For example, the spots can be analyzed using either MALDI or SELDI (*e.g.,* using ProteinChip^{®} array) as described herein.

Optionally, a marker can be modified before analysis to improve its resolution or to determine its identity. For example, the markers may be subject to proteolytic digestion before analysis. Any protease can be used. Proteases, such as trypsin, that are likely to cleave the markers into a discrete number of fragments are particularly useful. The fragments that result from digestion function as a fingerprint for the markers, thereby enabling their detection indirectly. This is particularly useful where there are markers with similar molecular masses that might be confused for the marker in question. Also, proteolytic fragmentation is useful for high molecular weight markers because smaller markers are more easily resolved by mass spectrometry. In another example, biomolecules can be modified to improve detection resolution. For instance, neuraminidase can be used to remove terminal sialic acid residues from glycoproteins to improve binding to an anionic adsorbent (*e.g.,* cationic exchange ProteinChip^{®} arrays) and to improve detection resolution. In another example, the markers can be modified by the attachment of a tag of particular molecular weight that specifically bind to molecular markers, further distinguishing them. Optionally, after detecting such modified markers, the identity of the markers can be further determined by matching the physical and chemical characteristics of the modified markers in a protein database (*e.g.,* SwissProt).

### III. CAPTURE OF MARKERS

Biomarkers are preferably captured with capture reagents immobilized to a solid support, such as any biochip described herein, a multiwell microtiter plate or a resin. In particular, the biomarkers of this invention are preferably captured on SELDI protein biochips. Capture can be on a chromatographic surface or a biospecific surface. Any of the SELDI protein biochips comprising reactive surfaces can be used to capture and detect the biomarkers of this invention. However, the biomarkers of this invention bind well to immobilized metal chelates. The IMAC-3 and IMAC 30 biochips, which nitriloacetic acid functionalities that adsorb transition metal ions, such as Cu⁺⁺ and Ni⁺⁺, by chelation, are the preferred SELDI biochips for capturing the biomarkers of this invention. Any of the SELDI protein biochips comprising reactive surfaces can be used to capture and detect the biomarkers of this invention. These biochips can be derivatized with the antibodies that specifically capture the biomarkers, or they can be derivatized with capture reagents, such as protein A or protein G that bind immunoglobulins. Then the biomarkers can be captured in solution using specific antibodies and the captured markers isolated on chip through the capture reagent.

In general, a sample containing the biomarkers, such as serum, is placed on the active surface of a biochip for a sufficient time to allow binding. Then, unbound molecules are washed from the surface using a suitable eluant, such as phosphate buffered saline. In general, the more stringent the eluant, the more tightly the proteins must be bound to be retained after the wash. The retained protein biomarkers now can be detected by appropriate means.

### IV. DETECTION AND MEASUREMENT OF MARKERS

Once captured on a substrate, e.g., biochip or antibody, any suitable method can be used to measure a marker or markers in a sample. For example, markers can be detected and/or measured by a variety of detection methods including for example, gas phase ion spectrometry methods, optical methods, electrochemical methods, atomic force microscopy and radio frequency methods. Using these methods, one or more markers can be detected.

### A) SELDI

One preferred method of detection and/or measurement of the biomarkers uses mass spectrometry and, in particular, "Surface-enhanced laser desorption/ionization" or "SELDI". SELDI refers to a method of desorption/ionization gas phase ion spectrometry (e.g., mass spectrometry) in which the analyte is captured on the surface of a SELDI probe that engages the probe interface. In "SELDI MS," the gas phase ion spectrometer is a mass spectrometer. SELDI technology is described in more detail above.

### B) IMMUNOASSAY

In another embodiment, an immunoassay can be used to detect and analyze markers in a sample. This method comprises: (a) providing an antibody that specifically binds to a marker; (b) contacting a sample with the antibody; and (c) detecting the presence of a complex of the antibody bound to the marker in the sample.

An immunoassay is an assay that uses an antibody to specifically bind an antigen (*e.g.,* a marker). The immunoassay is characterized by the use of specific binding properties of a particular antibody to isolate, target, and/or quantify the antigen. The phrase "specifically (or selectively) binds" to an antibody or "specifically (or selectively) immunoreactive with," when referring to a protein or peptide, refers to a binding reaction that is determinative of the presence of the protein in a heterogeneous population of proteins and other biologics. Thus, under designated immunoassay conditions, the specified antibodies bind to a particular protein at least two times the background and do not substantially bind in a significant amount to other proteins present in the sample. Specific binding to an antibody under such conditions may require an antibody that is selected for its specificity for a particular protein. For example, polyclonal antibodies raised to a marker from specific species such as rat, mouse, or human can be selected to obtain only those polyclonal antibodies that are specifically immunoreactive with that marker and not with other proteins, except for polymorphic variants and alleles of the marker. This selection may be achieved by subtracting out antibodies that cross-react with the marker molecules from other species.

Using the purified markers or their nucleic acid sequences, antibodies that specifically bind to a marker can be prepared using any suitable methods known in the art. *See, e.g.,* Coligan, Current Protocols in Immunology (1991); Harlow & Lane, Antibodies: A Laboratory Manual (1988); Goding, Monoclonal Antibodies: Principles and Practice (2d ed. 1986); and Kohler & Milstein, Nature 256:495-497 (1975). Such techniques include, but are not limited to, antibody preparation by selection of antibodies from libraries of recombinant antibodies in phage or similar vectors, as well as preparation of polyclonal and monoclonal antibodies by immunizing rabbits or mice (*see, e.g.,* Huse et al., Science 246:1275-1281 (1989); Ward et al., Nature 341:544-546 (1989)). Typically a specific or selective reaction will be at least twice background signal or noise and more typically more than 10 to 100 times background.

Generally, a sample obtained from a subject can be contacted with the antibody that specifically binds the marker. Optionally, the antibody can be fixed to a solid support to facilitate washing and subsequent isolation of the complex, prior to contacting the antibody with a sample. Examples of solid supports include glass or plastic in the form of, *e.g.,* a microtiter plate, a stick, a bead, or a microbead. Antibodies can also be attached to a probe substrate or ProteinChip^{®} array described above. The sample is preferably a biological fluid sample taken from a subject. Examples of biological fluid samples include blood, serum, plasma, nipple aspirate, urine, tears, saliva *etc.* In a preferred embodiment, the biological fluid comprises blood serum. The sample can be diluted with a suitable eluant before contacting the sample to the antibody.

After incubating the sample with antibodies, the mixture is washed and the antibody-marker complex formed can be detected. This can be accomplished by incubating the washed mixture with a detection reagent. This detection reagent may be, *e.g.,* a second antibody which is labeled with a detectable label. Exemplary detectable labels include magnetic beads (*e.g.,* DYNABEADS^{™}), fluorescent dyes, radiolabels, enzymes (*e.g.,* horse radish peroxide, alkaline phosphatase and others commonly used in an ELISA), and colorimetric labels such as colloidal gold or colored glass or plastic beads. Alternatively, the marker in the sample can be detected using an indirect assay, wherein, for example, a second, labeled antibody is used to detect bound marker-specific antibody, and/or in a competition or inhibition assay wherein, for example, a monoclonal antibody which binds to a distinct epitope of the marker is incubated simultaneously with the mixture.

Methods for measuring the amount of, or presence of, antibody-marker complex include, for example, detection of fluorescence, luminescence, chemiluminescence, absorbance, reflectance, transmittance, birefringence or refractive index (e.g., surface plasmon resonance, ellipsometry, a resonant mirror method, a grating coupler waveguide method or interferometry). Optical methods include microscopy (both confocal and non-confocal), imaging methods and non-imaging methods. Electrochemical methods include voltametry and amperometry methods. Radio frequency methods include multipolar resonance spectroscopy. Methods for performing these assays are readily known in the art. Useful assays include, for example, an enzyme immune assay (EIA) such as enzyme-linked immunosorbent assay (ELISA), a radioimmune assay (RIA), a Western blot assay, or a slot blot assay. These methods are also described in, *e.g.,* Methods in Cell Biology: Antibodies in Cell Biology, volume 37 (Asai, ed. 1993); Basic and Clinical Immunology (Stites & Terr, eds., 7th ed. 1991); and Harlow & Lane, *supra.*

Throughout the assays, incubation and/or washing steps may be required after each combination of reagents. Incubation steps can vary from about 5 seconds to several hours, preferably from about 5 minutes to about 24 hours. However, the incubation time will depend upon the assay format, marker, volume of solution, concentrations and the like. Usually the assays will be carried out at ambient temperature, although they can be conducted over a range of temperatures, such as 10°C to 40°C.

Immunoassays can be used to determine presence or absence of a marker in a sample as well as the quantity of a marker in a sample. The amount of an antibody-marker complex can be determined by comparing to a standard. A standard can be, e.g., a known compound or another protein known to be present in a sample. As noted above, the test amount of marker need not be measured in absolute units, as long as the unit of measurement can be compared to a control.

The methods for detecting these markers in a sample have many applications. For example, one or more markers can be measured to aid human cancer diagnosis or prognosis. In another example, the methods for detection of the markers can be used to monitor responses in a subject to cancer treatment. In another example, the methods for detecting markers can be used to assay for and to identify compounds that modulate expression of these markers *in vivo* or *in vitro.* In a preferred example, the biomarkers are used to differentiate between the different stages of tumor progression, thus aiding in determining appropriate treatment and extent of metastasis of the tumor.

### C) COMBINATORIAL LIGAND LIBRARY BEADS

Another method of measuring the biomarkers includes the use of a combinatorial ligand library synthesized on beads as described in USSN: 11/495,842, filed July 28, 2006 and entitled "Methods for Reducing the range in Concentrations of Analyte Species in a Sample".

### V. DATA ANALYSIS

When the sample is measured and data is generated, e.g., by mass spectrometry, the data is then analyzed by a computer software program. Generally, the software can comprise code that converts signal from the mass spectrometer into computer readable form. The software also can include code that applies an algorithm to the analysis of the signal to determine whether the signal represents a "peak" in the signal corresponding to a marker of this invention, or other useful markers. The software also can include code that executes an algorithm that compares signal from a test sample to a typical signal characteristic of "normal" and human cancer and determines the closeness of fit between the two signals. The software also can include code indicating which the test sample is closest to, thereby providing a probable diagnosis.

In preferred methods of the present invention, multiple biomarkers are measured. The use of multiple biomarkers increases the predictive value of the test and provides greater utility in diagnosis, toxicology, patient stratification and patient monitoring. The process called "Pattern recognition" detects the patterns formed by multiple biomarkers greatly improves the sensitivity and specificity of clinical proteomics for predictive medicine. Subtle variations in data from clinical samples, e.g., obtained using SELDI, indicate that certain patterns of protein expression can predict phenotypes such as the presence or absence of a certain disease, a particular stage of cancer progression, or a positive or adverse response to drug treatments.

Data generation in mass spectrometry begins with the detection of ions by an ion detector as described above. Ions that strike the detector generate an electric potential that is digitized by a high speed time-array recording device that digitally captures the analog signal. Ciphergen's ProteinChip^{®} system employs an analog-to-digital converter (ADC) to accomplish this. The ADC integrates detector output at regularly spaced time intervals into time-dependent bins. The time intervals typically are one to four nanoseconds long. Furthermore, the time-of-flight spectrum ultimately analyzed typically does not represent the signal from a single pulse of ionizing energy against a sample, but rather the sum of signals from a number of pulses. This reduces noise and increases dynamic range. This time-of-flight data is then subject to data processing. In Ciphergen's ProteinChip^{®} software, data processing typically includes TOF-to-M/Z transformation, baseline subtraction, high frequency noise filtering.

TOF-to-M/Z transformation involves the application of an algorithm that transforms times-of-flight into mass-to-charge ratio (M/Z). In this step, the signals are converted from the time domain to the mass domain. That is, each time-of-flight is converted into mass-to-charge ratio, or M/Z. Calibration can be done internally or externally. In internal calibration, the sample analyzed contains one or more analytes of known M/Z. Signal peaks at times-of-flight representing these massed analytes are assigned the known M/Z. Based on these assigned M/Z ratios, parameters are calculated for a mathematical function that converts times-of-flight to M/Z. In external calibration, a function that converts times-of-flight to M/Z, such as one created by prior internal calibration, is applied to a time-of-flight spectrum without the use of internal calibrants.

Baseline subtraction improves data quantification by eliminating artificial, reproducible instrument offsets that perturb the spectrum. It involves calculating a spectrum baseline using an algorithm that incorporates parameters such as peak width, and then subtracting the baseline from the mass spectrum.

High frequency noise signals are eliminated by the application of a smoothing function. A typical smoothing function applies a moving average function to each time-dependent bin. In an improved version, the moving average filter is a variable width digital filter in which the bandwidth of the filter varies as a function of, e.g., peak bandwidth, generally becoming broader with increased time-of-flight. See, e.g., WO 00/70648, November 23, 2000 (Gavin et al., "Variable Width Digital Filter for Time-of-flight Mass Spectrometry").

Analysis generally involves the identification of peaks in the spectrum that represent signal from an analyte. Peak selection can, of course, be done by eye. However, software is available as part of Ciphergen's ProteinChip® software that can automate the detection of peaks. In general, this software functions by identifying signals having a signal-to-noise ratio above a selected threshold and labeling the mass of the peak at the centroid of the peak signal. In one useful application many spectra are compared to identify identical peaks present in some selected percentage of the mass spectra. One version of this software clusters all peaks appearing in the various spectra within a defined mass range, and assigns a mass (M/Z) to all the peaks that are near the mid-point of the mass (M/Z) cluster.

Peak data from one or more spectra can be subject to further analysis by, for example, creating a spreadsheet in which each row represents a particular mass spectrum, each column represents a peak in the spectra defined by mass, and each cell includes the intensity of the peak in that particular spectrum. Various statistical or pattern recognition approaches can applied to the data.

In one example, Ciphergen's Biomarker Patterns^{™} Software is used to detect a pattern in the spectra that are generated. The data is classified using a pattern recognition process that uses a classification model. In general, the spectra will represent samples from at least two different groups for which a classification algorithm is sought. For example, the groups can be pathological v. non-pathological (e.g., cancer v. non-cancer), drug responder v. drug non-responder, toxic response v. non-toxic response, progressor to disease state v. non-progressor to disease state, phenotypic condition present v. phenotypic condition absent.

The spectra that are generated in embodiments of the invention can be classified using a pattern recognition process that uses a classification model. In some embodiments, data derived from the spectra (*e.g.,* mass spectra or time-of-flight spectra) that are generated using samples such as "known samples" can then be used to "train" a classification model. A "known sample" is a sample that is pre-classified (*e.g.,* cancer or not cancer). Data derived from the spectra (e.g., mass spectra or time-of-flight spectra) that are generated using samples such as "known samples" can then be used to "train" a classification model. A "known sample" is a sample that is pre-classified. The data that are derived from the spectra and are used to form the classification model can be referred to as a "training data set". Once trained, the classification model can recognize patterns in data derived from spectra generated using unknown samples. The classification model can then be used to classify the unknown samples into classes. This can be useful, for example, in predicting whether or not a particular biological sample is associated with a certain biological condition (e.g., diseased vs. non diseased).

The training data set that is used to form the classification model may comprise raw data or pre-processed data. In some embodiments, raw data can be obtained directly from time-of-flight spectra or mass spectra, and then may be optionally "pre-processed" in any suitable manner. For example, signals above a predetermined signal-to-noise ratio can be selected so that a subset of peaks in a spectrum is selected, rather than selecting all peaks in a spectrum. In another example, a predetermined number of peak "clusters" at a common value (*e.g.,* a particular time-of-flight value or mass-to-charge ratio value) can be used to select peaks. Illustratively, if a peak at a given mass-to-charge ratio is in less than 50% of the mass spectra in a group of mass spectra, then the peak at that mass-to-charge ratio can be omitted from the training data set. Pre-processing steps such as these can be used to reduce the amount of data that is used to train the classification model.

Classification models can be formed using any suitable statistical classification (or "learning") method that attempts to segregate bodies of data into classes based on objective parameters present in the data. Classification methods may be either supervised or unsupervised. Examples of supervised and unsupervised classification processes are described in Jain, "Statistical Pattern Recognition: A Review", IEEE Transactions on Pattern Analysis and Machine Intelligence, Vol. 22, No. 1, January 2000.

In supervised classification, training data containing examples of known categories are presented to a learning mechanism, which learns one more sets of relationships that define each of the known classes. New data may then be applied to the learning mechanism, which then classifies the new data using the learned relationships. Examples of supervised classification processes include linear regression processes (e.g., multiple linear regression (MLR), partial least squares (PLS) regression and principal components regression (PCR)), binary decision trees (e.g., recursive partitioning processes such as CART - classification and regression trees), artificial neural networks such as backpropagation networks, discriminant analyses (e.g., Bayesian classifier or Fischer analysis), logistic classifiers, and support vector classifiers (support vector machines).

A preferred supervised classification method is a recursive partitioning process. Recursive partitioning processes use recursive partitioning trees to classify spectra derived from unknown samples. Further details about recursive partitioning processes are provided in U.S. 2002 0138208 A1 (Paulse et al., "Method for analyzing mass spectra," September 26, 2002.

The classification models that are created can be formed using unsupervised learning methods. Unsupervised classification attempts to learn classifications based on similarities in the training data set, without pre classifying the spectra from which the training data set was derived. Unsupervised learning methods include cluster analyses. A cluster analysis attempts to divide the data into "clusters" or groups that ideally should have members that are very similar to each other, and very dissimilar to members of other clusters. Similarity is then measured using some distance metric, which measures the distance between data items, and clusters together data items that are closer to each other. Clustering techniques include the MacQueen's K-means algorithm and the Kohonen's Self-Organizing Map algorithm.

Learning algorithms asserted for use in classifying biological information are described in, for example, WO 01/31580 (Bamhill et al., "Methods and devices for identifying patterns in biological systems and methods of use thereof," May 3, 2001); U.S. 2002/0193950 A1 (Gavin et al., "Method or analyzing mass spectra," December 19, 2002); U.S. 2003/0004402 A1 (Hitt et al., "Process for discriminating between biological states based on hidden patterns from biological data," January 2, 2003); and U.S. Patent No.: 7,113,896 A1 (Zhang and Zhang, "Systems and methods for processing biological expression data" March 20, 2003).

More specifically, to obtain the biomarkers, the peak intensity data of samples from cancer patients and healthy controls were used as a "discovery set." This data were combined and randomly divided into a training set and a test set to construct and test multivariate predictive models.

Generally, the data generated from Section IV above is inputted into a diagnostic algorithm (i.e., classification algorithm as described above). The classification algorithm is then generated based on the learning algorithm. The process involves developing an algorithm that can generate the classification algorithm. The methods of the present invention generate a more accurate classification algorithm by accessing a number of ovarian cancer and normal samples of a sufficient number based on statistical sample calculations. The samples are used as a training set of data on learning algorithm.

The generation of the classification, i.e., diagnostic, algorithm is dependent upon the assay protocol used to analyze samples and generate the data obtained in Section IV above. It is imperative that the protocol for the detection and/or measurement of the markers (e.g., in step IV) must be the same as that used to obtain the data used for developing the classification algorithm. The assay conditions, which must be maintained throughout the training and classification systems include chip type and mass spectrometer parameters, as well as general protocols for sample preparation and testing. If the protocol for the detection and/or measurement of the markers (step IV) is changed, the learning algorithm and classification algorithm must also change. Similarly, if the learning algorithm and classification algorithm change, then the protocol for the detection and/or measurement of markers (step IV) must also change to be consistent with that used to generate classification algorithm. Development of a new classification model would require accessing a sufficient number of ovarian cancer and normal samples, developing a new training set of data based on a new detection protocol, generating a new classification algorithm using the data and finally, verifying the classification algorithm with a multi-site study.

The classification models can be formed on and used on any suitable digital computer. Suitable digital computers include micro, mini, or large computers using any standard or specialized operating system such as a Unix, Windows™ or Linux™ based operating system. The digital computer that is used may be physically separate from the mass spectrometer that is used to create the spectra of interest, or it may be coupled to the mass spectrometer. If it is separate from the mass spectrometer, the data must be inputted into the computer by some other means, whether manually or automated.

The training data set and the classification models according to embodiments of the invention can be embodied by computer code that is executed or used by a digital computer. The computer code can be stored on any suitable computer readable media including optical or magnetic disks, sticks, tapes, etc., and can be written in any suitable computer programming language including C, C++, visual basic, etc.

### VI. DIAGNOSIS OF SUBJECT AND DETERMINATION OF OVARIAN CANCER STATUS

This panel of biomarkers comparing (i) apolipoprotein A1 (ApoA1), (ii) transthyretin (TTR), (iii) inter-alpha (globulin) inhibitor H4 (plasma Kallikrein-sensitive glycoprotein) (ITIH4), (iv) transferrin (TFR) and (v) CA125 (referential), or (i) apolipoprotein A1 (ApoA1), (ii) transthyretin (TTR), (iii) CTAPIII, and (iv) CA125 (according to the invention), or respective fragment thereof, is useful in aiding in the determination of early stage ovarian cancer status. First, the selected biomarkers are measured in a subject sample using the methods described herein, e.g., capture on a SELDI biochip followed by detection by mass spectrometry. Then, the measurements is compared with a diagnostic amount or control that distinguishes an ovarian cancer status from a non-cancer status. The diagnostic amounts will reflect the information herein that the particular biomarkers are up-regulated or down-regulated in a cancer status compared with a non-cancer status. As is well understood in the art, the particular diagnostic amounts used can be adjusted to increase sensitivity or specificity of the diagnostic assay depending on the preference of the diagnostician. The test amounts as compared with the diagnostic amount thus indicates ovarian cancer status.

While individual biomarkers are useful diagnostic markers, it has been found that the panels of biomarkers described herein provide greater diagnostic value than single markers alone for differentiating between cancer free patients and patients with early stage, e.g., stage I or II ovarian cancer. Specifically, the detection of a plurality of markers in a sample increases the percentage of true positive and true negative diagnoses and would decrease the percentage of false positive or false negative diagnoses. Thus, method of the present invention comprises the measurement of the above biomarker. For example, the methods of the present invention have an AUC from ROC analysis greater than 0.50, more preferred methods have an AUC greater than 0.60, more preferred methods have an AUC greater than 0.70. Especially preferred methods have an AUC greater than 0.70 and most preferred methods have an AUC greater than 0.80.

The mere presence or absence of a marker detected with a detection cutoff, without quantifying the amount of marker, is useful and can be correlated with a probable diagnosis of ovarian cancer. For example, ITIH4 fragment can be more frequently detected in human ovarian cancer patients than in normal subjects. Equally, for example, biomarkers Apo A1 and transthyretin, can be less frequently detected in human ovarian cancer patients than in normal subjects. Also, CA125 is elevated in cancerous cells as compared to non-cancerous cells. Thus, a detected presence or absence, respectively, of these markers in a subject being tested indicates that the subject has a higher probability of having ovarian cancer.

The measurement of markers can involve quantifying the markers to correlate the detection of markers with a probable diagnosis of ovarian cancer. Thus, if the amount of the markers detected in a subject being tested is different compared to a control amount (i.e., higher or lower than the control, depending on the marker), then the subject being tested has a higher probability of having ovarian cancer.

The correlation may take into account the amount of the marker or markers in the sample compared to a control amount of the marker or markers (up or down regulation of the marker or markers) (*e.g.,* in normal subjects in whom human cancer is undetectable). A control can be, *e.g.,* the average or median amount of marker present in comparable samples of normal subjects in whom human cancer is undetectable. The control amount is measured under the same or substantially similar experimental conditions as in measuring the test amount. The correlation may take into account the presence or absence of the markers in a test sample and the frequency of detection of the same markers in a control. The correlation may take into account both of such factors to facilitate determination of ovarian cancer status.

In certain embodiments of the methods of qualifying early stage ovarian cancer status, the methods further comprise managing subject treatment based on the status. As aforesaid, such management describes the actions of the physician or clinician subsequent to determining ovarian cancer status. For example, if the result of the methods of the present invention is inconclusive or there is reason that confirmation of status is necessary, the physician may order more tests. Alternatively, if the status indicates that surgery is appropriate, the physician may schedule the patient for surgery. In other instances, the patient may receive chemotherapy or radiation treatments, either in lieu of, or in addition to, surgery. Likewise, if the result is negative, e.g., the status indicates late stage ovarian cancer or if the status is otherwise acute, no further action may be warranted. Furthermore, if the results show that treatment has been successful, no further management may be necessary.

Also described are methods where the claimed biomarkers are measured again after subject management. In these cases, the methods are used to monitor the status of the cancer, e.g., response to cancer treatment, remission of the disease or progression of the disease. Because of the ease of use of the methods and the lack of invasiveness of the methods, the methods can be repeated after each treatment the patient receives. This allows the physician to follow the effectiveness of the course of treatment. If the results show that the treatment is not effective, the course of treatment can be altered accordingly. This enables the physician to be flexible in the treatment options.

In another example, the methods for detecting markers can be used to assay for and to identify compounds that modulate expression of these markers *in vivo* or *in vitro.*

The methods of the present invention have other applications as well. For example, the markers can be used to screen for compounds that modulate the expression of the markers *in vitro* or *in vivo,* which compounds in turn may be useful in treating or preventing ovarian cancer in patients. In another example, the markers can be used to monitor the response to treatments for ovarian cancer. In yet another example, the markers can be used in heredity studies to determine if the subject is at risk for developing ovarian cancer. For instance, certain markers may be genetically linked. This can be determined by, *e.g.,* analyzing samples from a population of ovarian cancer patients whose families have a history of ovarian cancer. The results can then be compared with data obtained from, *e.g.,* ovarian cancer patients whose families do not have a history of ovarian cancer. The markers that are genetically linked may be used as a tool to determine if a subject whose family has a history of ovarian cancer is pre-disposed to having ovarian cancer.

### VIII. KITS

Described are kits for qualifying ovarian cancer status, wherein the kits can be used to measure the markers of the present invention. For example, the kits can be used to measure a panel of markers described herein, which markers are differentially present in samples of ovarian cancer patient and normal subjects. The kits have many applications. For example, the kits can be used to differentiate if a subject has ovarian cancer or has a negative diagnosis, thus enabling the physician or clinician to diagnose the presence or absence of the cancer. The kits can also be used to monitor the patient's response to a course of treatment, enabling the physician to modify the treatment based upon the results of the test. In another example, the kits can be used to identify compounds that modulate expression of one or more of the markers in *in vitro* or *in vivo* animal models for ovarian cancer.

Described are kits comprising (a) a capture reagent that binds a panel of biomarkers comprising (i) apolipoprotein A1 (ApoA1), (ii) transthyretin (TTR), (iii) inter-alpha (globulin) inhibitor H4 (plasma Kallikrein-sensitive glycoprotein) (ITIH4), (iv) transferrin (TFR) and (v) CA125, or (i) apolipoprotein A1 (ApoA1), (ii) transthyretin (TTR), (iii) CTAPIII, and (iv) CA125 or fragments thereof; and (b) a container comprising at least one of the biomarkers.

While the capture reagents can be any type of reagent, preferably the reagent is a SELDI probe. In certain kits, the capture reagent comprises an IMAC.

Described are also kits comprising (a) a capture reagents that bind the panel of biomarkers comprising (i) apolipoprotein A1 (ApoA1), (ii) transthyretin (TTR), (iii) inter-alpha (globulin) inhibitor H4 (plasma Kallikrein-sensitive glycoprotein) (ITIH4), (iv) transferrin (TFR) and (v) CA125, or (i) apolipoprotein A1 (ApoA1), (ii) transthyretin (TTR), (iii) CTAPIII, and (iv) CA125 and (b) instructions for using the capture reagent to measure the biomarker. In certain of these kits, the capture reagents comprise antibodies. Furthermore, some of the aforesaid kits further comprise an MS probe to which the capture reagent is attached or is attachable. In some kits, the capture reagent comprises an IMAC. One preferred embodiment of the present invention includes a high-throughput test for early detection of ovarian cancer, which analyzes a patient's sample on the IMAC ProteinChip^{®} array for the analytes.

Certain kits further comprise a wash solution, or eluant, that selectively allows retention of the bound biomarkers to the capture reagents as compared with other biomarkers after washing. Alternatively, the kit may contain instructions for making a wash solution, wherein the combination of the adsorbent and the wash solution allows detection of the markers using gas phase ion spectrometry.

Preferably, the kit comprises written instructions for use of the kit for detection of cancer and the instructions provide for contacting a test sample with the capture reagents and detecting the panel of biomarkers retained by the capture reagents. For example, the kit may have standard instructions informing a consumer how to wash the capture reagents (e.g., probes) after a sample of blood serum contacts the capture reagents. In another example, the kit may have instructions for pre-fractionating a sample to reduce complexity of proteins in the sample. In another example, the kit may have instructions for automating the fractionation or other processes.

Such kits can be prepared from the materials described above, and the previous discussion of these materials (e.g., probe substrates, capture reagents, adsorbents, washing solutions, *etc.*) is fully applicable to this section and will not be repeated.

In another embodiment, a kit comprises (a) antibodies that specifically bind to the panel of biomarkers; and (b) a detection reagent. Such kits can be prepared from the materials described above, and the previous discussion regarding the materials (*e.g.,* antibodies, detection reagents, immobilized supports, *etc.*) is fully applicable to this section and will not be repeated. Optionally, the kit may further comprise pre-fractionation spin columns. In some embodiments, the kit may further comprise instructions for suitable operation parameters in the form of a label or a separate insert.

Optionally, the kit may further comprise a standard or control information so that the test sample can be compared with the control information standard to determine if the test amount of a marker detected in a sample is a diagnostic amount consistent with a diagnosis of ovarian cancer.

Further described is an article manufacture comprising at least one capture reagent bound to at least two biomarkers selected from (i) apolipoprotein A1 (ApoA1), (ii) transthyretin (TTR), (iii) inter-alpha (globulin) inhibitor H4 (plasma Kallikrein-sensitive glycoprotein) (ITIH4), (iv) transferrin (TFR) and (v) CA125, or (i) apolipoprotein A1 (ApoA1), (ii) transthyretin (TTR), (iii) CTAPIII, and (iv) CA125. Examples of articles of manufacture include, but are not limited to, ProteinChip^{®} Arrays, probes, microtitre plates, beads, test tubes, microtubes, and any other solid phase onto which a capture reagent can be incorporated.

Described is also a system comprising a plurality of capture reagents each of which has bound to it a different biomarker comprising (i) apolipoprotein A1 (ApoA1), (ii) transthyretin (TTR), (iii) inter-alpha (globulin) inhibitor H4 (plasma Kallikrein-sensitive glycoprotein) (ITIH4), (iv) transferrin (TFR) and (v) CA125, or (i) apolipoprotein A1 (ApoA1), (ii) transthyretin (TTR), (iii) CTAPIII, and (iv) CA 125 or respective fragment thereof. Examples of other systems include those in which the capture reagents are test tubes containing an antibody for each of the biomarkers, either separately, or in groups. One of ordinary skill in the art would readily be able to manufacture other such articles in accordance with the teachings described herein.

The following examples are offered by way of illustration, not by way of limitation. While specific examples have been provided, the above description is illustrative and not restrictive. Any one or more of the features of the previously described embodiments can be combined in any manner with one or more features of any other embodiments in the present invention. Furthermore, many variations of the invention will become apparent to those skilled in the art upon review of the specification. The scope of the invention should, therefore, be determined not with reference to the above description, but instead should be determined with reference to the appended claims along with their full scope of equivalents.

### EXAMPLES

### Proteomic Markers Enhance the Sensitivity of CA 125 For Detection of Stage I Epithelial Ovarian Cancer

Given the prevalence of ovarian cancer, a successful strategy for the detection of early stage disease will require both a high sensitivity (>90%) and specificity (99.6%). The requisite specificity might be attained through a two stage strategy that utilizes an abnormal value for serum markers (s) to prompt the performance of transvaginal sonography (TVS). To perform TVS in a maximum of 2% of individuals screened, a specificity of 98% would be required. Greater sensitivity at 98% specificity than obtained with CA 125 alone might be achieved with the addition of proteomic biomarkers. The aim of this study was to test a panel of proteomic serum biomarkers for their ability to enhance CA125 in distinguishing individuals with early stage ovarian cancer from healthy subjects and from women with benign pelvic masses.

### Materials and Methods:

### Samples

We obtained pre-operative serum samples from the MDACC Ovarian Cancer Tumor Bank consisting of 92 patients with epithelial ovarian cancer (22 stage I, 19 stage II, 41 stage III and 10 stage IV), 40 with benign disease and 99 healthy individuals.

### Sample Measurements

Chromatographic SELDI-TOF-MS protocols have been developed to generate quantitative measurements. Standard curves were generated using known quantities of calibrants. Samples were processed in triplicate and randomized in 96 well bioprocessors using a Biomek2000 robot. Samples were processed using a Tecan Aquarius™ robot, and protocols performed using different ProteinChip® Arrays. Samples were diluted in respective buffers to assay. Transthyretin (TT), apolipoprotein, A (ApoA1), connective-tissue activating protein III (CTAPIII), inter-alpha trypsin inhibitor IV (internal fragment) (ITIH4), hepicidin (HepC), beta- 2 microglobulin (B2M) and transferrin. In the TT, ApoA1 and CTAPIII assays, E. coli lysate was mixed with the binding buffers. TT was assayed on Q10 arrays, ApoA1 was assayed on H50 arrays, CTAPIII and B2M were assayed together on IMAC50-Cu arrays, and ITIH4, HepC and transferrin were assayed together on IMAC50-Cu arrays. Arrays were analyzed on a PCS4000 mass spectrometer.

### Analysis

Peak intensity data were baseline subtracted and normalized using either total ion current factors or peak ratio factors. TIC normalized values were used for Hepcidin, ITIH4, Transferrin, and B2M. Ratio to average E coli peak intensity were used for CTAP3, TT ratio, and ApoA1. Models were fit using logistic regression, followed by a stepwise optimization of the factors retained in the models determined by optimizing the Akaike Information Criterion.

**Table 1. Description of patient samples**

| Sample Type | Number | Median Age (range) | Median CA125 (range) |
|---|---|---|---|
| Stage I | 22 | 49 (20-87) | 56 (8, 3 - 1273) |
| Stage II | 19 | 61 (41-88) | 190 (7-2000) |
| Stage III, IV | 51 | 57 (31-85) | 420 (6.5-26,000) |
| Benign | 40 | 45 (26-80) | 17 (2.1 - 189) |
| Normal | 99 | 62 (51-77) | 11 (3.9 - 73) |

### Results:

Two isoforms of transthyretin were combined to calculate the concentration of this marker. In the analysis of this patient population, it was found that subsets of the measured markers were sufficient for building the classification models.

| | |
|---|---|
| Markers | Mass |
| Transthyretin (cysteinylated form) | 13880.2 |
| Transthyretin (un-modified form) | 13841.0 |
| Apolipoprotein A1 | 28079.6 |
| ITIH4 fragment | 3273.7 |
| Transferrin | 79908 |
| Hepcidin-25 | 2790.4 |
| CTAPIII | 9288.7 |
| Beta 2-microglobin | 11731.2 |

Marker subsets used in Model development
Normal versus Stage I: ApoA1, TT, ITIH4, Transferrin, CA 125 (referential)
Normal versus Stages I, II: ApoA1, TT, CTAPIII, CA125 (invention)
Normal versus Stages I-IV: ApoA1, TT, CTAPIII, CA125 (referential)
Normal and Benign versus any cancer grouping: ApoA1, TT, CTAPIII, CA125 (referential)

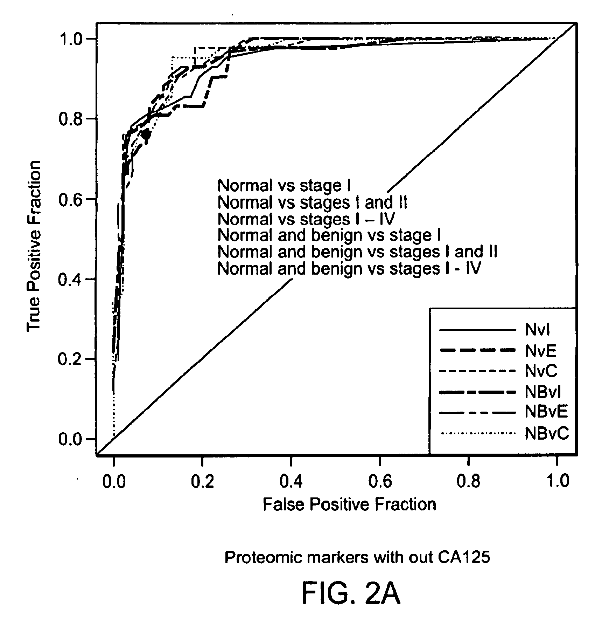

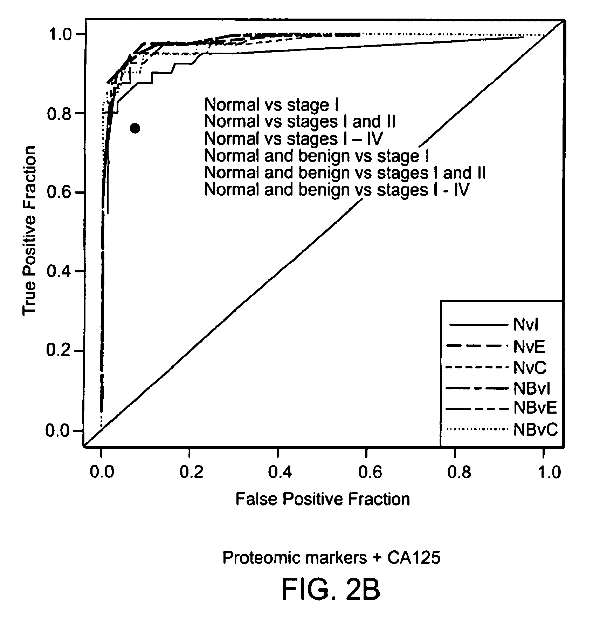

Figure 2 depicts ROC curve plots for models trained on six subsets of data. The black dot represents CA125 performance alone. Performance of the models, however, was always assessed by comparing normal controls to early stage (I or II) ovarian cancer. Thus, specificity refers to the ability of a model to correctly classify normal samples, and sensitivity refers to the ability of model to correctly classify early stage cancer.

**Table 3: Sensitivities of Models at 98% Specificity**

| A) CA125 Alone | | B) Proteomic Markers Alone |
|---|---|---|
| | | |

| Comparison | Sensitivity | Comparison |
|---|---|---|
| Normal versus stage I | 0.68 | Normal versus stage I |
| Normal versus stage I and II | 0.71 | Normal versus stage I and II |
| Normal versus stage I-IV | 0.76 | Normal versus stage I-IV |

| | C) Proteomic Markers + CA 125 | |
|---|---|---|
| | Comparison | Sensitivity |
| | | |
| | Normal versus stage I | 0.80 |
| | Normal versus stage I and II | **0.88** |
| | Normal versus stage I-IV | 0.85 |

Conclusion: A panel of proteomic biomarkers in combination with CA 125 improves detection of early stage ovarian cancer.

The present invention has been described in detail, including the preferred embodiments thereof.

## Claims

1. An in vitro method of determining if an ovarian cancer patient has an early stage of ovarian cancer comprising:
(a) determining the concentration or expression levels or peak intensity values of a combination of biomarkers in a blood, serum, or plasma sample from the subject, wherein the biomarkers consist of: (i) apolipoprotein A1 (ApoA1), (ii) transthyretin (TTR), (iii) CTAPIII, and (iv) CA125; and
(b) correlating the measurement of the combination of biomarkers of a) with ovarian cancer status, wherein the early stage ovarian cancer status is stage I or II ovarian cancer.

2. The method of claim 1 further comprising: (c) managing subject treatment based on the status.

3. The method of any one of claims 1 through 2, wherein the correlating is performed by a software classification algorithm.

## Patentansprüche

1. In-vitro-Verfahren zum Bestimmen, ob eine Ovarialkarzinom-Patientin ein Ovarialkarzinom im frühen Stadium hat, umfassend:
(a) Bestimmen der Konzentration oder der Expressionslevel oder der Peakintensitätswerte einer Kombination von Biomarkern in einer Blut-, Serum- oder Plasmaprobe von dem Subjekt, wobei die Biomarker aus: (i) Apolipoprotein A1 (ApoA1), (ii) Transthyretin (TTR), (iii) CTAPIII und (iv) CA125 bestehen, und
(b) Korrelieren der Messung der Kombination von Biomarkern von a) mit dem Ovarialkarzinom-Status, wobei der Status Ovarialkarzinom im frühen Stadium Ovarialkrebs Stadium I oder II ist.

2. Verfahren gemäß Anspruch 1, außerdem umfassend:
(c) Durchführen einer Behandlung des Subjektes auf der Basis des Status.

3. Verfahren gemäß einem der Ansprüche 1 bis 3, wobei das Korrelieren durch einen Software-Klassifizierungsalgorithmus durchgeführt wird.

## Revendications

1. Un procédé de détermination in vitro si un patient a un cancer de l'ovaire de stade précoce, comprenant:
(a) la détermination de la concentration ou des niveaux d'expression ou des valeurs de l'intensité de crête d'une combinaison de biomarqueurs dans un échantillon de sang, de sérum ou de plasma provenant du sujet, dans lequel les biomarqueurs consistant en (i) apolipoprotéine A1 (ApoAl), (ii) transthyrétine (TTR), (iii) CTAPIII et (iv) CA125; et
(b) la corrélation de la mesure de la combinaison de biomarqueurs de (a) avec l'état du cancer de l'ovaire, l'état d'un stade précoce du cancer de l'ovaire étant le cancer de l'ovaire de stade I ou de stade II.

2. Le procédé selon la revendication 1, comprenant en outre:
(c) la gestion du traitement de l'objet sur la base de l'état.

3. Le procédé selon l'une quelconque des revendications 1 à 2, dans lequel la corrélation est effectuée par un algorithme de classification de logiciel.
